# EUROPEAN PATENT APPLICATION

(11) **EP 2 123 768 A1**
(43) Date of publication of application: **25.11.2009**
(21) Application number: 08156600.2
(22) Date of filing: 20.05.2008
(51) Int. Cl.: C12P 17/10

(54) **Preparation of (Z)-6,7-dihydro-1H-azepin-2(5H)-one**

(71) Applicant: DSM IP Assets B.V., 6411 TE Heerlen (NL)
(72) Inventor: The designation of the inventor has not yet been filed
(74) Representative: van der Ploeg, Antonius Franciscus M.J.

(57) **Abstract**

The present invention relates to a method for preparing (*Z*)-6,7-dihydro-1*H*-azepin-2(5*H*)-one comprising removing the α-amino group of α-amino-ε-caprolactam, wherein the removal is catalysed by a biocatalyst. The invention further relates to a method for preparing caprolactam from (*Z*)-6,7-dihydro-1*H*-azepin-2(5*H*)-one. The invention further relates to a host cell comprising at least one recombinant vector comprising a nucleic acid sequence encoding a biocatalyst with L-α-amino-ε-caprolactam ammonia lyase activity.

SEQUENCE LISTING

<110> DSM IP ASSETS B.V.

<120> Preparation of (Z)-6,7-dihydro-1H-azepin-2(5H)-one

<130> P84471EP00

<160> 18

<170> PatentIn version 3.5

<210> 1
<211> 1212
<212> DNA
<213> Candida macedoniensis

<220>
<221> CDS
<222> (1)..(1212)

<400> 1
atg tcg tac atg aac ttt gac cct aag cca ttg gga gac acc aat atc 48
Met Ser Tyr Met Asn Phe Asp Pro Lys Pro Leu Gly Asp Thr Asn Ile
1               5                   10                  15

ttc aag cca atc aag atc ggt aac aat gag cta aaa cac aga gta gtc 96
Phe Lys Pro Ile Lys Ile Gly Asn Asn Glu Leu Lys His Arg Val Val
            20                  25                  30

atg cca gca ttg act aga atg aga gcc att gca cca gga aac atc cca 144
Met Pro Ala Leu Thr Arg Met Arg Ala Ile Ala Pro Gly Asn Ile Pro
        35                  40                  45

aac act gaa tgg gcc gag gaa tac tac aga caa cgt tct caa tac cct 192
Asn Thr Glu Trp Ala Glu Glu Tyr Tyr Arg Gln Arg Ser Gln Tyr Pro
    50                  55                  60

ggt acc ctt att atc acg gaa ggt act ttc cct tct gcg caa tca ggt 240
Gly Thr Leu Ile Ile Thr Glu Gly Thr Phe Pro Ser Ala Gln Ser Gly
65                  70                  75                  80

ggt tac cca aat gtg cca ggt atc tgg tcc aaa gag caa ttg gct gaa 288
Gly Tyr Pro Asn Val Pro Gly Ile Trp Ser Lys Glu Gln Leu Ala Glu
                85                  90                  95

tgg aaa aag atc ttc aat gca atc cat gag aac aaa tcg ttc gtg tgg 336
Trp Lys Lys Ile Phe Asn Ala Ile His Glu Asn Lys Ser Phe Val Trp
            100                 105                 110

gtg caa ttg tgg gtt cta ggt aga caa gca tgg cca gaa gtg ttg aag 384
Val Gln Leu Trp Val Leu Gly Arg Gln Ala Trp Pro Glu Val Leu Lys
        115                 120                 125

aag gaa ggt ttg cgt tac gat agt gct acc gat gac ttg tac atg ggt 432
Lys Glu Gly Leu Arg Tyr Asp Ser Ala Thr Asp Asp Leu Tyr Met Gly

    130                 135                 140

gaa gaa gaa aaa gag cgt gcc tta aag gct aac aac cca cag cac ggt 480
Glu Glu Glu Lys Glu Arg Ala Leu Lys Ala Asn Asn Pro Gln His Gly
145                 150                 155                 160

atc acc aag gaa gaa atc aag cag tac atc aag gag tac gtg gat gct 528
Ile Thr Lys Glu Glu Ile Lys Gln Tyr Ile Lys Glu Tyr Val Asp Ala
                165                 170                 175

gcc aag aaa gcc atc gat gca ggt gca gac ggt gtg caa atc cat tct 576
Ala Lys Lys Ala Ile Asp Ala Gly Ala Asp Gly Val Gln Ile His Ser
            180                 185                 190

gcc aac ggt tac ttg ttg aac cag ttt ttg gac cct att tct aac aac 624
Ala Asn Gly Tyr Leu Leu Asn Gln Phe Leu Asp Pro Ile Ser Asn Asn
        195                 200                 205

aga acc gac gag tac ggt gga tcg atc gag aac cgt gcg aga ttc act 672
Arg Thr Asp Glu Tyr Gly Gly Ser Ile Glu Asn Arg Ala Arg Phe Thr
    210                 215                 220

ttg gaa gtg gtc gat gcc gtt gtc gat gca gtt ggt gcc gaa aga acc 720
Leu Glu Val Val Asp Ala Val Val Asp Ala Val Gly Ala Glu Arg Thr
225                 230                 235                 240

tcc atc aga ttc tct cca tac ggt act ttt ggt acc atg tcc ggt ggt 768
Ser Ile Arg Phe Ser Pro Tyr Gly Thr Phe Gly Thr Met Ser Gly Gly
                245                 250                 255

gag aac cct ggc atc gtt gct caa tat gca tac gtc att ggt gag ttg 816
Glu Asn Pro Gly Ile Val Ala Gln Tyr Ala Tyr Val Ile Gly Glu Leu
            260                 265                 270

gaa aag aga gct aga gct ggc aag aga ttg gcg ttc atc gat ttg gtc 864
Glu Lys Arg Ala Arg Ala Gly Lys Arg Leu Ala Phe Ile Asp Leu Val
        275                 280                 285

gag cct cgt gtg acc gac cca ttc cta cca gaa ttc gag aag tgg ttc 912
Glu Pro Arg Val Thr Asp Pro Phe Leu Pro Glu Phe Glu Lys Trp Phe
    290                 295                 300

aag gaa ggt acc aac gaa ttc atc tac tct atc tgg aag ggt cca gtt 960
Lys Glu Gly Thr Asn Glu Phe Ile Tyr Ser Ile Trp Lys Gly Pro Val
305                 310                 315                 320

ctc aga gtt ggt aac tat gct ttg gac cca gat caa gcc act ctc gac 1008
Leu Arg Val Gly Asn Tyr Ala Leu Asp Pro Asp Gln Ala Thr Leu Asp
                325                 330                 335

tct aag aag cct aac act ttg atc ggt tac ggt aga tcc ttc atc gcc 1056
Ser Lys Lys Pro Asn Thr Leu Ile Gly Tyr Gly Arg Ser Phe Ile Ala
            340                 345                 350

aac cca gac ttg gtg tac cgt ttg gaa aag ggt ttg cca ttg aac aag 1104
Asn Pro Asp Leu Val Tyr Arg Leu Glu Lys Gly Leu Pro Leu Asn Lys
        355                 360                 365

tat gat aga aac acc ttt tac aca ttc act aag gaa ggt tac acc gat      1152
Tyr Asp Arg Asn Thr Phe Tyr Thr Phe Thr Lys Glu Gly Tyr Thr Asp
   370                 375                 380

tac cca agc tac gaa gaa tcc gtc gca aag ggt tac aag aaa gag gaa      1200
Tyr Pro Ser Tyr Glu Glu Ser Val Ala Lys Gly Tyr Lys Lys Glu Glu
385                 390                 395                 400

aag aag tac taa                                                      1212
Lys Lys Tyr

<210> 2
<211> 403
<212> PRT
<213> Candida macedoniensis

<400> 2

Met Ser Tyr Met Asn Phe Asp Pro Lys Pro Leu Gly Asp Thr Asn Ile
1               5                   10                  15

Phe Lys Pro Ile Lys Ile Gly Asn Asn Glu Leu Lys His Arg Val Val
            20                  25                  30

Met Pro Ala Leu Thr Arg Met Arg Ala Ile Ala Pro Gly Asn Ile Pro
        35                  40                  45

Asn Thr Glu Trp Ala Glu Glu Tyr Tyr Arg Gln Arg Ser Gln Tyr Pro
    50                  55                  60

Gly Thr Leu Ile Ile Thr Glu Gly Thr Phe Pro Ser Ala Gln Ser Gly
65                  70                  75                  80

Gly Tyr Pro Asn Val Pro Gly Ile Trp Ser Lys Glu Gln Leu Ala Glu
                85                  90                  95

Trp Lys Lys Ile Phe Asn Ala Ile His Glu Asn Lys Ser Phe Val Trp
            100                 105                 110

Val Gln Leu Trp Val Leu Gly Arg Gln Ala Trp Pro Glu Val Leu Lys
        115                 120                 125

Lys Glu Gly Leu Arg Tyr Asp Ser Ala Thr Asp Asp Leu Tyr Met Gly
    130                 135                 140

Glu Glu Glu Lys Glu Arg Ala Leu Lys Ala Asn Asn Pro Gln His Gly
145                 150                 155                 160

Ile Thr Lys Glu Glu Ile Lys Gln Tyr Ile Lys Glu Tyr Val Asp Ala
            165                 170                 175

Ala Lys Lys Ala Ile Asp Ala Gly Ala Asp Gly Val Gln Ile His Ser
        180                 185                 190

Ala Asn Gly Tyr Leu Leu Asn Gln Phe Leu Asp Pro Ile Ser Asn Asn
    195                 200                 205

Arg Thr Asp Glu Tyr Gly Gly Ser Ile Glu Asn Arg Ala Arg Phe Thr
  210                 215                 220

Leu Glu Val Val Asp Ala Val Val Asp Ala Val Gly Ala Glu Arg Thr
225                 230                 235                 240

Ser Ile Arg Phe Ser Pro Tyr Gly Thr Phe Gly Thr Met Ser Gly Gly
            245                 250                 255

Glu Asn Pro Gly Ile Val Ala Gln Tyr Ala Tyr Val Ile Gly Glu Leu
        260                 265                 270

Glu Lys Arg Ala Arg Ala Gly Lys Arg Leu Ala Phe Ile Asp Leu Val
      275                 280                 285

Glu Pro Arg Val Thr Asp Pro Phe Leu Pro Glu Phe Glu Lys Trp Phe
  290                 295                 300

Lys Glu Gly Thr Asn Glu Phe Ile Tyr Ser Ile Trp Lys Gly Pro Val
305                 310                 315                 320

Leu Arg Val Gly Asn Tyr Ala Leu Asp Pro Asp Gln Ala Thr Leu Asp
            325                 330                 335

Ser Lys Lys Pro Asn Thr Leu Ile Gly Tyr Gly Arg Ser Phe Ile Ala
        340                 345                 350

Asn Pro Asp Leu Val Tyr Arg Leu Glu Lys Gly Leu Pro Leu Asn Lys
      355                 360                 365

Tyr Asp Arg Asn Thr Phe Tyr Thr Phe Thr Lys Glu Gly Tyr Thr Asp

    370                 375                 380

Tyr Pro Ser Tyr Glu Glu Ser Val Ala Lys Gly Tyr Lys Lys Glu Glu
385                 390                 395                 400

Lys Lys Tyr

<210> 3
<211> 1197
<212> DNA
<213> Kluyveromyces lactis

<220>
<221> CDS
<222> (1)..(1197)

<400> 3
atg tcg ttt atg aac ttt gaa cca aag cca ttg gct gat act gat atc 48
Met Ser Phe Met Asn Phe Glu Pro Lys Pro Leu Ala Asp Thr Asp Ile
1               5                   10                  15

ttc aaa cca atc aag att ggt aac act gaa ttg aag cac agg gtt gtc 96
Phe Lys Pro Ile Lys Ile Gly Asn Thr Glu Leu Lys His Arg Val Val
            20                  25                  30

atg cct gca ttg aca aga atg aga gcg ttg cat cca ggc aac gtt cca 144
Met Pro Ala Leu Thr Arg Met Arg Ala Leu His Pro Gly Asn Val Pro
        35                  40                  45

aac cct gac tgg gct gtt gaa tat tac aga caa cgt tcc caa tat cca 192
Asn Pro Asp Trp Ala Val Glu Tyr Tyr Arg Gln Arg Ser Gln Tyr Pro
    50                  55                  60

ggt act atg att atc act gaa ggt gct ttc cca tca gct cag tca ggt 240
Gly Thr Met Ile Ile Thr Glu Gly Ala Phe Pro Ser Ala Gln Ser Gly
65                  70                  75                  80

ggt tac gat aac gca cca ggt gtt tgg agc gaa gaa caa ctg gct caa 288
Gly Tyr Asp Asn Ala Pro Gly Val Trp Ser Glu Glu Gln Leu Ala Gln
                85                  90                  95

tgg aga aag atc ttc aag gca att cac gac aac aag tct ttt gtt tgg 336
Trp Arg Lys Ile Phe Lys Ala Ile His Asp Asn Lys Ser Phe Val Trp
            100                 105                 110

gta caa ttg tgg gtt cta ggt aga caa gct ttt gct gat aac ttg gca 384
Val Gln Leu Trp Val Leu Gly Arg Gln Ala Phe Ala Asp Asn Leu Ala
        115                 120                 125

aga gat gga ttg cgt tat gat agt gct tcc gat gaa gtg tac atg ggt 432
Arg Asp Gly Leu Arg Tyr Asp Ser Ala Ser Asp Glu Val Tyr Met Gly
    130                 135                 140

gaa gat gaa aag gaa cgt gcc atc aga tct aac aac cct cag cat ggt 480
Glu Asp Glu Lys Glu Arg Ala Ile Arg Ser Asn Asn Pro Gln His Gly
145                 150                 155                 160

atc acc aag gat gaa att aag cag tat atc agg gac tat gtt gat gct 528
Ile Thr Lys Asp Glu Ile Lys Gln Tyr Ile Arg Asp Tyr Val Asp Ala
                165                 170                 175

gct aag aag tgt atc gat gct ggt gca gat ggt gtt gaa atc cat tcc 576
Ala Lys Lys Cys Ile Asp Ala Gly Ala Asp Gly Val Glu Ile His Ser
            180                 185                 190

gct aac ggt tat ttg ttg aat caa ttc cta gac cca atc tcc aac aaa 624
Ala Asn Gly Tyr Leu Leu Asn Gln Phe Leu Asp Pro Ile Ser Asn Lys
        195                 200                 205

aga act gat gaa tac ggt gga tcc att gag aac cgt gct aga ttc gtc 672
Arg Thr Asp Glu Tyr Gly Gly Ser Ile Glu Asn Arg Ala Arg Phe Val
    210                 215                 220

ttg gaa gtc gtc gat gcc gtt gtc gat gcc gtt ggt gcc gaa aga acc 720
Leu Glu Val Val Asp Ala Val Val Asp Ala Val Gly Ala Glu Arg Thr
225                 230                 235                 240

agt atc aga ttc tca cca tac ggt gta ttt ggt acc atg tca ggt gtt 768
Ser Ile Arg Phe Ser Pro Tyr Gly Val Phe Gly Thr Met Ser Gly Val
                245                 250                 255

tca gac cct gtc ttg gtg gct caa ttc gcc tat gta ctt gct gaa ttg 816
Ser Asp Pro Val Leu Val Ala Gln Phe Ala Tyr Val Leu Ala Glu Leu
            260                 265                 270

gaa aag agg gca aag gct ggt aag aga tta gca tac gtc gat tta gtc 864
Glu Lys Arg Ala Lys Ala Gly Lys Arg Leu Ala Tyr Val Asp Leu Val
        275                 280                 285

gaa cct cgt gtc aca tcg cca ttc caa ccg gaa ttt gaa ggc tgg tat 912
Glu Pro Arg Val Thr Ser Pro Phe Gln Pro Glu Phe Glu Gly Trp Tyr
    290                 295                 300

aaa ggt ggt acc aat gaa ttc gta tac tct gtt tgg aag ggt aac gtg 960
Lys Gly Gly Thr Asn Glu Phe Val Tyr Ser Val Trp Lys Gly Asn Val
305                 310                 315                 320

cta aga gtt ggt aac tac gct ttg gac cca gat gct gcc att acg gac 1008
Leu Arg Val Gly Asn Tyr Ala Leu Asp Pro Asp Ala Ala Ile Thr Asp
                325                 330                 335

tca aag aat cca aac act ttg atc ggt tac ggt aga gcc ttc att gcc 1056
Ser Lys Asn Pro Asn Thr Leu Ile Gly Tyr Gly Arg Ala Phe Ile Ala
            340                 345                 350

aac cca gat ctt gtt gaa cgt ctc gaa aag ggt ttg cca ttg aat caa 1104
Asn Pro Asp Leu Val Glu Arg Leu Glu Lys Gly Leu Pro Leu Asn Gln
        355                 360                 365

tac gat aga ccc tct ttc tac aaa atg tct gcg gaa ggg tat atc gac      1152
Tyr Asp Arg Pro Ser Phe Tyr Lys Met Ser Ala Glu Gly Tyr Ile Asp
   370                 375                 380

tac cca aca tac gag gaa gct gtt gcc aag ggt tac aag aaa tag          1197
Tyr Pro Thr Tyr Glu Glu Ala Val Ala Lys Gly Tyr Lys Lys
385                 390                 395

<210>  4
<211>  398
<212>  PRT
<213>  Kluyveromyces lactis

<400>  4

Met Ser Phe Met Asn Phe Glu Pro Lys Pro Leu Ala Asp Thr Asp Ile
1               5                   10                  15

Phe Lys Pro Ile Lys Ile Gly Asn Thr Glu Leu Lys His Arg Val Val
            20                  25                  30

Met Pro Ala Leu Thr Arg Met Arg Ala Leu His Pro Gly Asn Val Pro
        35                  40                  45

Asn Pro Asp Trp Ala Val Glu Tyr Tyr Arg Gln Arg Ser Gln Tyr Pro
    50                  55                  60

Gly Thr Met Ile Ile Thr Glu Gly Ala Phe Pro Ser Ala Gln Ser Gly
65                  70                  75                  80

Gly Tyr Asp Asn Ala Pro Gly Val Trp Ser Glu Glu Gln Leu Ala Gln
                85                  90                  95

Trp Arg Lys Ile Phe Lys Ala Ile His Asp Asn Lys Ser Phe Val Trp
            100                 105                 110

Val Gln Leu Trp Val Leu Gly Arg Gln Ala Phe Ala Asp Asn Leu Ala
        115                 120                 125

Arg Asp Gly Leu Arg Tyr Asp Ser Ala Ser Asp Glu Val Tyr Met Gly
    130                 135                 140

Glu Asp Glu Lys Glu Arg Ala Ile Arg Ser Asn Asn Pro Gln His Gly
145                 150                 155                 160

Ile Thr Lys Asp Glu Ile Lys Gln Tyr Ile Arg Asp Tyr Val Asp Ala

                165                 170                 175

Ala Lys Lys Cys Ile Asp Ala Gly Ala Asp Gly Val Glu Ile His Ser
            180                 185                 190

Ala Asn Gly Tyr Leu Leu Asn Gln Phe Leu Asp Pro Ile Ser Asn Lys
        195                 200                 205

Arg Thr Asp Glu Tyr Gly Gly Ser Ile Glu Asn Arg Ala Arg Phe Val
    210                 215                 220

Leu Glu Val Val Asp Ala Val Val Asp Ala Val Gly Ala Glu Arg Thr
225                 230                 235                 240

Ser Ile Arg Phe Ser Pro Tyr Gly Val Phe Gly Thr Met Ser Gly Val
                245                 250                 255

Ser Asp Pro Val Leu Val Ala Gln Phe Ala Tyr Val Leu Ala Glu Leu
            260                 265                 270

Glu Lys Arg Ala Lys Ala Gly Lys Arg Leu Ala Tyr Val Asp Leu Val
        275                 280                 285

Glu Pro Arg Val Thr Ser Pro Phe Gln Pro Glu Phe Glu Gly Trp Tyr
    290                 295                 300

Lys Gly Gly Thr Asn Glu Phe Val Tyr Ser Val Trp Lys Gly Asn Val
305                 310                 315                 320

Leu Arg Val Gly Asn Tyr Ala Leu Asp Pro Asp Ala Ala Ile Thr Asp
                325                 330                 335

Ser Lys Asn Pro Asn Thr Leu Ile Gly Tyr Gly Arg Ala Phe Ile Ala
            340                 345                 350

Asn Pro Asp Leu Val Glu Arg Leu Glu Lys Gly Leu Pro Leu Asn Gln
        355                 360                 365

Tyr Asp Arg Pro Ser Phe Tyr Lys Met Ser Ala Glu Gly Tyr Ile Asp
    370                 375                 380

Tyr Pro Thr Tyr Glu Glu Ala Val Ala Lys Gly Tyr Lys Lys
385                 390                 395

<210> 5
<211> 1050
<212> DNA
<213> Pseudomonas fluorescens

<220>
<221> CDS
<222> (1)..(1050)

<400> 5
atg gca act att ttc gat ccg atc aaa ctg ggc gac ctc gag ctg tcc 48
Met Ala Thr Ile Phe Asp Pro Ile Lys Leu Gly Asp Leu Glu Leu Ser
1               5                   10                  15

aac cgc atc atc atg gcc ccg ctg act cgc tgc cgc gcc gac gaa ggc 96
Asn Arg Ile Ile Met Ala Pro Leu Thr Arg Cys Arg Ala Asp Glu Gly
            20                  25                  30

cgc gta ccc aac gca ctg atg gcc gag tac tac gtg caa cgt gcc tcc 144
Arg Val Pro Asn Ala Leu Met Ala Glu Tyr Tyr Val Gln Arg Ala Ser
        35                  40                  45

gcc ggc ctg att ctc agc gaa gcc act tcg gtg acg ccg atg ggc gtc 192
Ala Gly Leu Ile Leu Ser Glu Ala Thr Ser Val Thr Pro Met Gly Val
    50                  55                  60

ggc tat ccg gac acc ccg ggc atc tgg tcc aac gat cag gta cgc ggc 240
Gly Tyr Pro Asp Thr Pro Gly Ile Trp Ser Asn Asp Gln Val Arg Gly
65                  70                  75                  80

tgg acc aac atc acc aaa gcc gta cac gct gcc ggc ggc aag atc gtc 288
Trp Thr Asn Ile Thr Lys Ala Val His Ala Ala Gly Gly Lys Ile Val
                85                  90                  95

ctg caa ctt tgg cac gtc ggc cgc atc tcg cac ccg ttg tac ctg aac 336
Leu Gln Leu Trp His Val Gly Arg Ile Ser His Pro Leu Tyr Leu Asn
            100                 105                 110

ggc gaa gca ccg gtc gcg ccg agc gcc atc cag cct aaa ggc cac gtc 384
Gly Glu Ala Pro Val Ala Pro Ser Ala Ile Gln Pro Lys Gly His Val
        115                 120                 125

agc ctg gtg cgt cca ctg gcc gat tac ccg act cca cgc gcc ctg gaa 432
Ser Leu Val Arg Pro Leu Ala Asp Tyr Pro Thr Pro Arg Ala Leu Glu
    130                 135                 140

acc gct gaa atc gcc gag atc gtc gag gcc tac cgc acc ggt gcc gag 480
Thr Ala Glu Ile Ala Glu Ile Val Glu Ala Tyr Arg Thr Gly Ala Glu
145                 150                 155                 160

aac gcc aag gcc gcc ggt ttc gac ggc gtg gaa atc cac ggc gcc aac 528
Asn Ala Lys Ala Ala Gly Phe Asp Gly Val Glu Ile His Gly Ala Asn
                165                 170                 175

ggc tac ctg ctc gac cag ttc ttg caa agc agc acc aac cag cgc acc 576
Gly Tyr Leu Leu Asp Gln Phe Leu Gln Ser Ser Thr Asn Gln Arg Thr
            180                 185                 190

gac aat tac ggc ggc tcc ctg gaa aac cgt gcg cgt ctg ttg ctg gaa 624
Asp Asn Tyr Gly Gly Ser Leu Glu Asn Arg Ala Arg Leu Leu Leu Glu
        195                 200                 205

gtg act gat gcc gcg atc gac gtc tgg ggc gcc ggc cgt gtc ggt gtg 672
Val Thr Asp Ala Ala Ile Asp Val Trp Gly Ala Gly Arg Val Gly Val
    210                 215                 220

cac ctg gca ccg cgc gcc gac tcc cac gac atg ggc gac gac aac ctc 720
His Leu Ala Pro Arg Ala Asp Ser His Asp Met Gly Asp Asp Asn Leu
225                 230                 235                 240

gcc gag acc ttc acc tat gtt gct cgc gag ctg ggc aag cgt ggc atc 768
Ala Glu Thr Phe Thr Tyr Val Ala Arg Glu Leu Gly Lys Arg Gly Ile
                245                 250                 255

gcc ttc atc tgc tcc cgc gag aaa gaa ggc gcc gac agc ctc ggc cca 816
Ala Phe Ile Cys Ser Arg Glu Lys Glu Gly Ala Asp Ser Leu Gly Pro
            260                 265                 270

caa ctg aaa gaa gcc ttt ggc ggc gcg tac atc gcc aac gag cgt ttc 864
Gln Leu Lys Glu Ala Phe Gly Gly Ala Tyr Ile Ala Asn Glu Arg Phe
        275                 280                 285

acc aag gac agc gcc aat gcg tgg ctg gct gaa ggc aag gct gac gct 912
Thr Lys Asp Ser Ala Asn Ala Trp Leu Ala Glu Gly Lys Ala Asp Ala
    290                 295                 300

gta gcg ttc ggc gtg cca ttc att gcc aac ccg gac ctg ccg gca cgc 960
Val Ala Phe Gly Val Pro Phe Ile Ala Asn Pro Asp Leu Pro Ala Arg
305                 310                 315                 320

ctg aaa gcc gat gcc ccg ctg aac gag ccg cgt cct gag ctg ttc tat 1008
Leu Lys Ala Asp Ala Pro Leu Asn Glu Pro Arg Pro Glu Leu Phe Tyr
                325                 330                 335

ggc aaa ggc ccg gtc ggc tac atc gac tac ccg acg ctg taa 1050
Gly Lys Gly Pro Val Gly Tyr Ile Asp Tyr Pro Thr Leu
            340                 345

<210> 6
<211> 349
<212> PRT
<213> Pseudomonas fluorescens

<400> 6

Met Ala Thr Ile Phe Asp Pro Ile Lys Leu Gly Asp Leu Glu Leu Ser
1               5                   10                  15

Asn Arg Ile Ile Met Ala Pro Leu Thr Arg Cys Arg Ala Asp Glu Gly

            20                  25                  30

Arg Val Pro Asn Ala Leu Met Ala Glu Tyr Tyr Val Gln Arg Ala Ser
        35                  40                  45

Ala Gly Leu Ile Leu Ser Glu Ala Thr Ser Val Thr Pro Met Gly Val
    50                  55                  60

Gly Tyr Pro Asp Thr Pro Gly Ile Trp Ser Asn Asp Gln Val Arg Gly
65                  70                  75                  80

Trp Thr Asn Ile Thr Lys Ala Val His Ala Ala Gly Gly Lys Ile Val
85 90 . 95

Leu Gln Leu Trp His Val Gly Arg Ile Ser His Pro Leu Tyr Leu Asn
100 105 110

Gly Glu Ala Pro Val Ala Pro Ser Ala Ile Gln Pro Lys Gly His Val
115 120 125

Ser Leu Val Arg Pro Leu Ala Asp Tyr Pro Thr Pro Arg Ala Leu Glu
130 135 140

Thr Ala Glu Ile Ala Glu Ile Val Glu Ala Tyr Arg Thr Gly Ala Glu
145 150 155 160

Asn Ala Lys Ala Ala Gly Phe Asp Gly Val Glu Ile His Gly Ala Asn
165 170 175

Gly Tyr Leu Leu Asp Gln Phe Leu Gln Ser Ser Thr Asn Gln Arg Thr
180 185 190

Asp Asn Tyr Gly Gly Ser Leu Glu Asn Arg Ala Arg Leu Leu Leu Glu
195 200 205

Val Thr Asp Ala Ala Ile Asp Val Trp Gly Ala Gly Arg Val Gly Val
210 215 220

His Leu Ala Pro Arg Ala Asp Ser His Asp Met Gly Asp Asp Asn Leu
225 230 235 240

Ala Glu Thr Phe Thr Tyr Val Ala Arg Glu Leu Gly Lys Arg Gly Ile
245 250 255

Ala Phe Ile Cys Ser Arg Glu Lys Glu Gly Ala Asp Ser Leu Gly Pro
260 265 270

Gln Leu Lys Glu Ala Phe Gly Gly Ala Tyr Ile Ala Asn Glu Arg Phe
275 280 285

Thr Lys Asp Ser Ala Asn Ala Trp Leu Ala Glu Gly Lys Ala Asp Ala
290 295 300

Val Ala Phe Gly Val Pro Phe Ile Ala Asn Pro Asp Leu Pro Ala Arg
305 310 315 320

Leu Lys Ala Asp Ala Pro Leu Asn Glu Pro Arg Pro Glu Leu Phe Tyr
325 330 335

Gly Lys Gly Pro Val Gly Tyr Ile Asp Tyr Pro Thr Leu
340 345

<210> 7
<211> 1083
<212> DNA
<213> Pseudomonas syringae

<220>
<221> CDS
<222> (1)..(1083)

<400> 7
atg ccg act ctt ttc gac ccc ttg act ttg ggc gac ctg caa tct cca 48
Met Pro Thr Leu Phe Asp Pro Leu Thr Leu Gly Asp Leu Gln Ser Pro
1               5                   10                  15

aac cgt gtt ctg atg gca ccg cta acg cgt ggc cgc gcg acc cgc gag 96
Asn Arg Val Leu Met Ala Pro Leu Thr Arg Gly Arg Ala Thr Arg Glu
            20                  25                  30

cac gtg cct acc gag ctg atg atc gag tat tac acc cag cgt gcc agc 144
His Val Pro Thr Glu Leu Met Ile Glu Tyr Tyr Thr Gln Arg Ala Ser
        35                  40                  45

gcg ggc ctg atc atc acc gaa gcc acc ggc atc acc caa gaa ggc cta 192
Ala Gly Leu Ile Ile Thr Glu Ala Thr Gly Ile Thr Gln Glu Gly Leu
    50                  55                  60

ggc tgg ccc tat gcg ccc ggc att tgg agc gat gaa cag gtc gag gcc 240
Gly Trp Pro Tyr Ala Pro Gly Ile Trp Ser Asp Glu Gln Val Glu Ala
65                  70                  75                  80

tgg aag ccg gtg acc cag gcc gtg cat gag gca ggc gga cgg atc att 288
Trp Lys Pro Val Thr Gln Ala Val His Glu Ala Gly Gly Arg Ile Ile
                85                  90                  95

ctt cag ttg tgg cat atg ggc cgt acc gtt cat tcc agc ttt ctc ggc 336
Leu Gln Leu Trp His Met Gly Arg Thr Val His Ser Ser Phe Leu Gly
            100                 105                 110

gga gcc aag cca gta tcg tcc tcg gcc acc cgt gcg ccg gga cag gcg 384
Gly Ala Lys Pro Val Ser Ser Ser Ala Thr Arg Ala Pro Gly Gln Ala
        115                 120                 125

cac acc tac gaa ggc aag caa gac tac gac gag gcg cgg cct ttg tcg 432
His Thr Tyr Glu Gly Lys Gln Asp Tyr Asp Glu Ala Arg Pro Leu Ser
    130                 135                 140

gcg gat gaa atc ccg cgg cta ttg aac gat tac gaa cac gca gcg aaa 480
Ala Asp Glu Ile Pro Arg Leu Leu Asn Asp Tyr Glu His Ala Ala Lys
145                 150                 155                 160

aac gcc atg gcc gca ggc ttc gac ggc gtg cag atc cat gct gcc aat 528
Asn Ala Met Ala Ala Gly Phe Asp Gly Val Gln Ile His Ala Ala Asn
                165                 170                 175

ggt tac cta atc gac cag ttc ctg cgc gac aac agc aac gtt cgc ggg 576
Gly Tyr Leu Ile Asp Gln Phe Leu Arg Asp Asn Ser Asn Val Arg Gly
            180                 185                 190

gac gcc tac ggg ggt tca atc gag aac cgc atc cgt cta ttg gtc gaa 624
Asp Ala Tyr Gly Gly Ser Ile Glu Asn Arg Ile Arg Leu Leu Val Glu
        195                 200                 205

gtc acc cgg cgc gtg gcg gag acc gta ggt gcc gaa aaa acg ggc gtg 672
Val Thr Arg Arg Val Ala Glu Thr Val Gly Ala Glu Lys Thr Gly Val
    210                 215                 220

cgg ctg tca ccc aac ggt gat tcc caa ggc gtc aac gac agc aat ccg 720
Arg Leu Ser Pro Asn Gly Asp Ser Gln Gly Val Asn Asp Ser Asn Pro
225                 230                 235                 240

gag ccg ctg ttc agc gcc gcg gcc aag gcc ttg gat gag atc ggc atc 768
Glu Pro Leu Phe Ser Ala Ala Ala Lys Ala Leu Asp Glu Ile Gly Ile
                245                 250                 255

gct cat ctg gag ttg cgc gaa cca ggg tat gaa ggc acc ttc ggc aag 816
Ala His Leu Glu Leu Arg Glu Pro Gly Tyr Glu Gly Thr Phe Gly Lys
            260                 265                 270

gcc gac cgg ccg ccc gtg cac ccg gtc atc cgc cag gcg ttc agc cgt 864
Ala Asp Arg Pro Pro Val His Pro Val Ile Arg Gln Ala Phe Ser Arg
        275                 280                 285

acg ctg att ctc aac tct gac tac act ttg gaa acg gct cag gct gca 912
Thr Leu Ile Leu Asn Ser Asp Tyr Thr Leu Glu Thr Ala Gln Ala Ala
    290                 295                 300

cta gcc acc gga gaa gcg gac gcg atc acc ttc ggc cgc ccg ttc ctg 960

Leu Ala Thr Gly Glu Ala Asp Ala Ile Thr Phe Gly Arg Pro Phe Leu
305                 310                 315                 320

gcc aac cct gac ctg cct cac agg ttt gcc gag aga ctg ccg ctg aac       1008
Ala Asn Pro Asp Leu Pro His Arg Phe Ala Glu Arg Leu Pro Leu Asn
                325                 330                 335

aag gac gtg atg gag act tgg tat agc cag ggg ccc gaa ggt tat gtg       1056
Lys Asp Val Met Glu Thr Trp Tyr Ser Gln Gly Pro Glu Gly Tyr Val
            340                 345                 350

gac tac ccc acc gct gac caa aag tag                                   1083
Asp Tyr Pro Thr Ala Asp Gln Lys
        355                 360

<210>  8
<211>  360
<212>  PRT
<213>  Pseudomonas syringae

<400>  8

Met Pro Thr Leu Phe Asp Pro Leu Thr Leu Gly Asp Leu Gln Ser Pro
1               5                   10                  15

Asn Arg Val Leu Met Ala Pro Leu Thr Arg Gly Arg Ala Thr Arg Glu
            20                  25                  30

His Val Pro Thr Glu Leu Met Ile Glu Tyr Tyr Thr Gln Arg Ala Ser
        35                  40                  45

Ala Gly Leu Ile Ile Thr Glu Ala Thr Gly Ile Thr Gln Glu Gly Leu
    50                  55                  60

Gly Trp Pro Tyr Ala Pro Gly Ile Trp Ser Asp Glu Gln Val Glu Ala
65                  70                  75                  80

Trp Lys Pro Val Thr Gln Ala Val His Glu Ala Gly Gly Arg Ile Ile
                85                  90                  95

Leu Gln Leu Trp His Met Gly Arg Thr Val His Ser Ser Phe Leu Gly
            100                 105                 110

Gly Ala Lys Pro Val Ser Ser Ser Ala Thr Arg Ala Pro Gly Gln Ala
        115                 120                 125

His Thr Tyr Glu Gly Lys Gln Asp Tyr Asp Glu Ala Arg Pro Leu Ser
    130                 135                 140

Ala Asp Glu Ile Pro Arg Leu Leu Asn Asp Tyr Glu His Ala Ala Lys
145                 150                 155                 160

Asn Ala Met Ala Ala Gly Phe Asp Gly Val Gln Ile His Ala Ala Asn
                165                 170                 175

Gly Tyr Leu Ile Asp Gln Phe Leu Arg Asp Asn Ser Asn Val Arg Gly
            180                 185                 190

Asp Ala Tyr Gly Gly Ser Ile Glu Asn Arg Ile Arg Leu Leu Val Glu
        195                 200                 205

Val Thr Arg Arg Val Ala Glu Thr Val Gly Ala Glu Lys Thr Gly Val
    210                 215                 220

Arg Leu Ser Pro Asn Gly Asp Ser Gln Gly Val Asn Asp Ser Asn Pro
225                 230                 235                 240

Glu Pro Leu Phe Ser Ala Ala Ala Lys Ala Leu Asp Glu Ile Gly Ile
                245                 250                 255

Ala His Leu Glu Leu Arg Glu Pro Gly Tyr Glu Gly Thr Phe Gly Lys
            260                 265                 270

Ala Asp Arg Pro Pro Val His Pro Val Ile Arg Gln Ala Phe Ser Arg
        275                 280                 285

Thr Leu Ile Leu Asn Ser Asp Tyr Thr Leu Glu Thr Ala Gln Ala Ala
    290                 295                 300

Leu Ala Thr Gly Glu Ala Asp Ala Ile Thr Phe Gly Arg Pro Phe Leu
305                 310                 315                 320

Ala Asn Pro Asp Leu Pro His Arg Phe Ala Glu Arg Leu Pro Leu Asn
                325                 330                 335

Lys Asp Val Met Glu Thr Trp Tyr Ser Gln Gly Pro Glu Gly Tyr Val
            340                 345                 350

Asp Tyr Pro Thr Ala Asp Gln Lys
        355                 360

<210> 9
<211> 1098
<212> DNA
<213> Escherichia coli

<220>
<221> CDS
<222> (1)..(1098)

<400> 9
atg tca tct gaa aaa ctg tat tcc cca ctg aaa gtg ggc gcg atc acg 48
Met Ser Ser Glu Lys Leu Tyr Ser Pro Leu Lys Val Gly Ala Ile Thr
1               5                   10                  15

gcg gca aac cgt att ttt atg gca ccg ctg acg cgt ctg cgc agt att 96
Ala Ala Asn Arg Ile Phe Met Ala Pro Leu Thr Arg Leu Arg Ser Ile
            20                  25                  30

gaa ccg ggt gac att cct acc ccg ttg atg gcg gaa tac tat cgc caa 144
Glu Pro Gly Asp Ile Pro Thr Pro Leu Met Ala Glu Tyr Tyr Arg Gln
        35                  40                  45

cgt gcc agt gcc ggt ttg att att agt gaa gcc acg caa att tct gcc 192
Arg Ala Ser Ala Gly Leu Ile Ile Ser Glu Ala Thr Gln Ile Ser Ala
    50                  55                  60

cag gca aaa gga tat gca ggt gcg cct ggc atc cat agt ccg gag caa 240
Gln Ala Lys Gly Tyr Ala Gly Ala Pro Gly Ile His Ser Pro Glu Gln
65                  70                  75                  80

att gcc gca tgg aaa aaa atc acc gct ggc gtt cat gct gaa aat ggt 288
Ile Ala Ala Trp Lys Lys Ile Thr Ala Gly Val His Ala Glu Asn Gly
                85                  90                  95

cat atg gcc gtg cag ctg tgg cac acc gga cgc att tct cac gcc agc 336
His Met Ala Val Gln Leu Trp His Thr Gly Arg Ile Ser His Ala Ser
            100                 105                 110

ctg caa cct ggc ggt cag gca ccg gta gcg cct tca gca ctt agc gcg 384
Leu Gln Pro Gly Gly Gln Ala Pro Val Ala Pro Ser Ala Leu Ser Ala
        115                 120                 125

gga aca cgt act tct ctg cgc gat gaa aat ggt cag gcg atc cgt gtt 432
Gly Thr Arg Thr Ser Leu Arg Asp Glu Asn Gly Gln Ala Ile Arg Val
    130                 135                 140

gaa aca tcc atg ccg cgt gcg ctt gaa ctg gaa gag att cca ggt atc 480
Glu Thr Ser Met Pro Arg Ala Leu Glu Leu Glu Glu Ile Pro Gly Ile
145                 150                 155                 160

gtc aat gat ttc cgt cag gcc att gct aac gcg cgt gaa gcc ggt ttt 528
Val Asn Asp Phe Arg Gln Ala Ile Ala Asn Ala Arg Glu Ala Gly Phe
                165                 170                 175

gat ctg gta gag ctc cac tct gct cac ggt tat ttg ctg cat cag ttc 576

Asp Leu Val Glu Leu His Ser Ala His Gly Tyr Leu Leu His Gln Phe
            180                 185                 190

ctt tct cct tct tca aac cat cgt acc gat cag tac ggc ggc agc gtg 624
Leu Ser Pro Ser Ser Asn His Arg Thr Asp Gln Tyr Gly Gly Ser Val
        195                 200                 205

gaa aat cgc gca cgt ttg gta ctg gaa gtg gtc gat gcc ggg att gaa 672
Glu Asn Arg Ala Arg Leu Val Leu Glu Val Val Asp Ala Gly Ile Glu
    210                 215                 220

gaa tgg ggt gcc gat cgc att ggc att cgc gtt tca cca atc ggt act 720
Glu Trp Gly Ala Asp Arg Ile Gly Ile Arg Val Ser Pro Ile Gly Thr
225                 230                 235                 240

ttc cag aac aca gat aac ggc ccg aat gaa gaa gcc gat gca ctg tat 768
Phe Gln Asn Thr Asp Asn Gly Pro Asn Glu Glu Ala Asp Ala Leu Tyr
                245                 250                 255

ctg att gaa caa ctg ggt aaa cgc ggc att gct tat ctg cat atg tca 816
Leu Ile Glu Gln Leu Gly Lys Arg Gly Ile Ala Tyr Leu His Met Ser
            260                 265                 270

gaa cca gat tgg gcg ggg ggt gaa ccg tat act gat gcg ttc cgc gaa 864
Glu Pro Asp Trp Ala Gly Gly Glu Pro Tyr Thr Asp Ala Phe Arg Glu
        275                 280                 285

aaa gta cgc gcc cgt ttc cac ggt ccg att atc ggc gca ggt gca tac 912
Lys Val Arg Ala Arg Phe His Gly Pro Ile Ile Gly Ala Gly Ala Tyr
    290                 295                 300

aca gta gaa aaa gct gaa acg ctg atc ggc aaa ggg tta att gat gcg 960
Thr Val Glu Lys Ala Glu Thr Leu Ile Gly Lys Gly Leu Ile Asp Ala
305                 310                 315                 320

gtg gca ttt ggt cgt gac tgg att gcg aac ccg gat ctg gtc gcc cgc 1008
Val Ala Phe Gly Arg Asp Trp Ile Ala Asn Pro Asp Leu Val Ala Arg
                325                 330                 335

ttg cag cgc aaa gct gag ctt aac cca cag cgt gcc gaa agt ttc tac 1056
Leu Gln Arg Lys Ala Glu Leu Asn Pro Gln Arg Ala Glu Ser Phe Tyr
            340                 345                 350

ggt ggc ggc gcg gaa ggc tat acc gat tac ccg acg ttg taa 1098
Gly Gly Gly Ala Glu Gly Tyr Thr Asp Tyr Pro Thr Leu
        355                 360                 365

<210> 10
<211> 365
<212> PRT
<213> Escherichia coli

<400> 10

Met Ser Ser Glu Lys Leu Tyr Ser Pro Leu Lys Val Gly Ala Ile Thr
1               5                   10                  15

Ala Ala Asn Arg Ile Phe Met Ala Pro Leu Thr Arg Leu Arg Ser Ile
            20                  25                  30

Glu Pro Gly Asp Ile Pro Thr Pro Leu Met Ala Glu Tyr Tyr Arg Gln
        35                  40                  45

Arg Ala Ser Ala Gly Leu Ile Ile Ser Glu Ala Thr Gln Ile Ser Ala
    50                  55                  60

Gln Ala Lys Gly Tyr Ala Gly Ala Pro Gly Ile His Ser Pro Glu Gln
65                  70                  75                  80

Ile Ala Ala Trp Lys Lys Ile Thr Ala Gly Val His Ala Glu Asn Gly
                85                  90                  95

His Met Ala Val Gln Leu Trp His Thr Gly Arg Ile Ser His Ala Ser
            100                 105                 110

Leu Gln Pro Gly Gly Gln Ala Pro Val Ala Pro Ser Ala Leu Ser Ala
        115                 120                 125

Gly Thr Arg Thr Ser Leu Arg Asp Glu Asn Gly Gln Ala Ile Arg Val
    130                 135                 140

Glu Thr Ser Met Pro Arg Ala Leu Glu Leu Glu Glu Ile Pro Gly Ile
145                 150                 155                 160

Val Asn Asp Phe Arg Gln Ala Ile Ala Asn Ala Arg Glu Ala Gly Phe
                165                 170                 175

Asp Leu Val Glu Leu His Ser Ala His Gly Tyr Leu Leu His Gln Phe
            180                 185                 190

Leu Ser Pro Ser Ser Asn His Arg Thr Asp Gln Tyr Gly Gly Ser Val
        195                 200                 205

Glu Asn Arg Ala Arg Leu Val Leu Glu Val Val Asp Ala Gly Ile Glu
    210                 215                 220

Glu Trp Gly Ala Asp Arg Ile Gly Ile Arg Val Ser Pro Ile Gly Thr
225                 230                 235                 240

Phe Gln Asn Thr Asp Asn Gly Pro Asn Glu Glu Ala Asp Ala Leu Tyr
                245                 250                 255

Leu Ile Glu Gln Leu Gly Lys Arg Gly Ile Ala Tyr Leu His Met Ser
            260                 265                 270

Glu Pro Asp Trp Ala Gly Gly Glu Pro Tyr Thr Asp Ala Phe Arg Glu
        275                 280                 285

Lys Val Arg Ala Arg Phe His Gly Pro Ile Ile Gly Ala Gly Ala Tyr
    290                 295                 300

Thr Val Glu Lys Ala Glu Thr Leu Ile Gly Lys Gly Leu Ile Asp Ala
305                 310                 315                 320

Val Ala Phe Gly Arg Asp Trp Ile Ala Asn Pro Asp Leu Val Ala Arg
                325                 330                 335

Leu Gln Arg Lys Ala Glu Leu Asn Pro Gln Arg Ala Glu Ser Phe Tyr
            340                 345                 350

Gly Gly Gly Ala Glu Gly Tyr Thr Asp Tyr Pro Thr Leu
        355                 360                 365

<210> 11
<211> 1203
<212> DNA
<213> Saccharomyces cerevisiae

<220>
<221> CDS
<222> (1)..(1203)

<400> 11
atg cca ttt gtt aag gac ttt aag cca caa gct ttg ggt gac acc aac 48
Met Pro Phe Val Lys Asp Phe Lys Pro Gln Ala Leu Gly Asp Thr Asn
1               5                   10                  15

tta ttc aaa cca atc aaa att ggt aac aat gaa ctt cta cac cgt gct 96
Leu Phe Lys Pro Ile Lys Ile Gly Asn Asn Glu Leu Leu His Arg Ala
            20                  25                  30

gtc att cct cca ttg act aga atg aga gcc caa cat cca ggt aat att 144
Val Ile Pro Pro Leu Thr Arg Met Arg Ala Gln His Pro Gly Asn Ile
        35                  40                  45

cca aac aga gac tgg gcc gtt gaa tac tac gct caa cgt gct caa aga 192

Pro Asn Arg Asp Trp Ala Val Glu Tyr Tyr Ala Gln Arg Ala Gln Arg
50                  55                  60

cca gga acc ttg att atc act gaa ggt acc ttt ccc tct cca caa tct        240
Pro Gly Thr Leu Ile Ile Thr Glu Gly Thr Phe Pro Ser Pro Gln Ser
65                  70                  75                  80

ggg ggt tac gac aat gct cca ggt atc tgg tcc gaa gaa caa att aaa        288
Gly Gly Tyr Asp Asn Ala Pro Gly Ile Trp Ser Glu Glu Gln Ile Lys
                85                  90                  95

gaa tgg acc aag att ttc aag gct att cat gag aat aaa tcg ttc gca        336
Glu Trp Thr Lys Ile Phe Lys Ala Ile His Glu Asn Lys Ser Phe Ala
            100                 105                 110

tgg gtc caa tta tgg gtt cta ggt tgg gct gct ttc cca gac acc ctt        384
Trp Val Gln Leu Trp Val Leu Gly Trp Ala Ala Phe Pro Asp Thr Leu
        115                 120                 125

gct agg gat ggt ttg cgt tac gac tcc gct tct gac aac gtg tat atg        432
Ala Arg Asp Gly Leu Arg Tyr Asp Ser Ala Ser Asp Asn Val Tyr Met
    130                 135                 140

aat gca gaa caa gaa gaa aag gct aag aag gct aac aac cca caa cac        480
Asn Ala Glu Gln Glu Glu Lys Ala Lys Lys Ala Asn Asn Pro Gln His
145                 150                 155                 160

agt ata aca aag gat gaa att aag caa tac gtc aaa gaa tac gtc caa        528
Ser Ile Thr Lys Asp Glu Ile Lys Gln Tyr Val Lys Glu Tyr Val Gln
                165                 170                 175

gct gcc aaa aac tcc att gct gct ggt gcc gat ggt gtt gaa atc cac        576
Ala Ala Lys Asn Ser Ile Ala Ala Gly Ala Asp Gly Val Glu Ile His
            180                 185                 190

agc gct aac ggt tac ttg ttg aac cag ttc ttg gac cca cac tcc aat        624
Ser Ala Asn Gly Tyr Leu Leu Asn Gln Phe Leu Asp Pro His Ser Asn
        195                 200                 205

aac aga acc gat gag tat ggt gga tcc atc gaa aac aga gcc cgt ttc        672
Asn Arg Thr Asp Glu Tyr Gly Gly Ser Ile Glu Asn Arg Ala Arg Phe
    210                 215                 220

acc ttg gaa gtg gtt gat gca gtt gtc gat gct att ggc cct gaa aaa        720
Thr Leu Glu Val Val Asp Ala Val Val Asp Ala Ile Gly Pro Glu Lys
225                 230                 235                 240

gtc ggt ttg aga ttg tct cca tat ggt gtc ttc aac agt atg tct ggt        768
Val Gly Leu Arg Leu Ser Pro Tyr Gly Val Phe Asn Ser Met Ser Gly
                245                 250                 255

ggt gct gaa acc ggt att gtt gct caa tat gct tat gtc tta ggt gaa        816
Gly Ala Glu Thr Gly Ile Val Ala Gln Tyr Ala Tyr Val Leu Gly Glu
            260                 265                 270

cta gaa aga aga gct aaa gct ggc aag cgt ttg gct ttc gtc cat cta        864
Leu Glu Arg Arg Ala Lys Ala Gly Lys Arg Leu Ala Phe Val His Leu

        275                 280                 285

gtt gaa cct cgt gtc acc aac cca ttt tta act gaa ggt gaa ggt gaa 912
Val Glu Pro Arg Val Thr Asn Pro Phe Leu Thr Glu Gly Glu Gly Glu
    290                 295                 300

tac aat gga ggt agc aac aaa ttt gct tat tct atc tgg aag ggc cca 960
Tyr Asn Gly Gly Ser Asn Lys Phe Ala Tyr Ser Ile Trp Lys Gly Pro
305                 310                 315                 320

att att aga gct ggt aac ttt gct ctg cac cca gaa gtt gtc aga gaa 1008
Ile Ile Arg Ala Gly Asn Phe Ala Leu His Pro Glu Val Val Arg Glu
                325                 330                 335

gag gtg aag gat cct aga aca ttg atc ggt tac ggt aga ttt ttt atc 1056
Glu Val Lys Asp Pro Arg Thr Leu Ile Gly Tyr Gly Arg Phe Phe Ile
            340                 345                 350

tct aat cca gat ttg gtt gat cgt ttg gaa aaa ggg tta cca tta aac 1104
Ser Asn Pro Asp Leu Val Asp Arg Leu Glu Lys Gly Leu Pro Leu Asn
        355                 360                 365

aaa tat gac aga gac act ttc tac aaa atg tca gct gag gga tac att 1152
Lys Tyr Asp Arg Asp Thr Phe Tyr Lys Met Ser Ala Glu Gly Tyr Ile
    370                 375                 380

gac tac cct acg tac gaa gaa gct cta aaa ctc ggt tgg gac aaa aat 1200
Asp Tyr Pro Thr Tyr Glu Glu Ala Leu Lys Leu Gly Trp Asp Lys Asn
385                 390                 395                 400

taa 1203

<210> 12
<211> 400
<212> PRT
<213> Saccharomyces cerevisiae

<400> 12

Met Pro Phe Val Lys Asp Phe Lys Pro Gln Ala Leu Gly Asp Thr Asn
1               5                   10                  15

Leu Phe Lys Pro Ile Lys Ile Gly Asn Asn Glu Leu Leu His Arg Ala
            20                  25                  30

Val Ile Pro Pro Leu Thr Arg Met Arg Ala Gln His Pro Gly Asn Ile
        35                  40                  45

Pro Asn Arg Asp Trp Ala Val Glu Tyr Tyr Ala Gln Arg Ala Gln Arg
    50                  55                  60

Pro Gly Thr Leu Ile Ile Thr Glu Gly Thr Phe Pro Ser Pro Gln Ser

65                70                75                80

Gly Gly Tyr Asp Asn Ala Pro Gly Ile Trp Ser Glu Glu Gln Ile Lys
            85                90                95

Glu Trp Thr Lys Ile Phe Lys Ala Ile His Glu Asn Lys Ser Phe Ala
        100               105               110

Trp Val Gln Leu Trp Val Leu Gly Trp Ala Ala Phe Pro Asp Thr Leu
      115               120               125

Ala Arg Asp Gly Leu Arg Tyr Asp Ser Ala Ser Asp Asn Val Tyr Met
   130               135               140

Asn Ala Glu Gln Glu Glu Lys Ala Lys Lys Ala Asn Asn Pro Gln His
145               150               155               160

Ser Ile Thr Lys Asp Glu Ile Lys Gln Tyr Val Lys Glu Tyr Val Gln
            165               170               175

Ala Ala Lys Asn Ser Ile Ala Ala Gly Ala Asp Gly Val Glu Ile His
        180               185               190

Ser Ala Asn Gly Tyr Leu Leu Asn Gln Phe Leu Asp Pro His Ser Asn
      195               200               205

Asn Arg Thr Asp Glu Tyr Gly Gly Ser Ile Glu Asn Arg Ala Arg Phe
   210               215               220

Thr Leu Glu Val Val Asp Ala Val Val Asp Ala Ile Gly Pro Glu Lys
225               230               235               240

Val Gly Leu Arg Leu Ser Pro Tyr Gly Val Phe Asn Ser Met Ser Gly
            245               250               255

Gly Ala Glu Thr Gly Ile Val Ala Gln Tyr Ala Tyr Val Leu Gly Glu
        260               265               270

Leu Glu Arg Arg Ala Lys Ala Gly Lys Arg Leu Ala Phe Val His Leu
      275               280               285

Val Glu Pro Arg Val Thr Asn Pro Phe Leu Thr Glu Gly Glu Gly Glu
   290               295               300

Tyr Asn Gly Gly Ser Asn Lys Phe Ala Tyr Ser Ile Trp Lys Gly Pro
305                 310                 315                 320

Ile Ile Arg Ala Gly Asn Phe Ala Leu His Pro Glu Val Val Arg Glu
                325                 330                 335

Glu Val Lys Asp Pro Arg Thr Leu Ile Gly Tyr Gly Arg Phe Phe Ile
            340                 345                 350

Ser Asn Pro Asp Leu Val Asp Arg Leu Glu Lys Gly Leu Pro Leu Asn
        355                 360                 365

Lys Tyr Asp Arg Asp Thr Phe Tyr Lys Met Ser Ala Glu Gly Tyr Ile
    370                 375                 380

Asp Tyr Pro Thr Tyr Glu Glu Ala Leu Lys Leu Gly Trp Asp Lys Asn
385                 390                 395                 400

<210>  13
<211>  1017
<212>  DNA
<213>  Bacillus subtilis

<220>
<221>  CDS
<222>  (1)..(1017)

<400>  13
atg gcc aga aaa tta ttt aca cct att aca att aaa gat atg acg tta        48
Met Ala Arg Lys Leu Phe Thr Pro Ile Thr Ile Lys Asp Met Thr Leu
1               5                   10                  15

aaa aac cgc att gtc atg tcg cca atg tgc atg tat tct tct cat gaa        96
Lys Asn Arg Ile Val Met Ser Pro Met Cys Met Tyr Ser Ser His Glu
            20                  25                  30

aag gac gga aaa tta aca ccg ttc cac atg gca cat tac ata tcg cgc        144
Lys Asp Gly Lys Leu Thr Pro Phe His Met Ala His Tyr Ile Ser Arg
        35                  40                  45

gca atc ggc cag gtc gga ctg att att gta gag gcg tca gcg gtt aac        192
Ala Ile Gly Gln Val Gly Leu Ile Ile Val Glu Ala Ser Ala Val Asn
    50                  55                  60

cct caa gga cga atc act gac caa gac tta ggc att tgg agc gac gag        240
Pro Gln Gly Arg Ile Thr Asp Gln Asp Leu Gly Ile Trp Ser Asp Glu
65                  70                  75                  80

cat att gaa ggc ttt gca aaa ctg act gag cag gtc aaa gaa caa ggt 288
His Ile Glu Gly Phe Ala Lys Leu Thr Glu Gln Val Lys Glu Gln Gly
                85                  90                  95

tca aaa atc ggc att cag ctt gcc cat gcc gga cgt aaa gct gag ctt 336
Ser Lys Ile Gly Ile Gln Leu Ala His Ala Gly Arg Lys Ala Glu Leu
            100                 105                 110

gaa gga gat atc ttc gct cca tcg gcg att gcg ttt gac gaa caa tca 384
Glu Gly Asp Ile Phe Ala Pro Ser Ala Ile Ala Phe Asp Glu Gln Ser
        115                 120                 125

gca aca cct gta gaa atg tca gca gaa aaa gta aaa gaa acg gtc cag 432
Ala Thr Pro Val Glu Met Ser Ala Glu Lys Val Lys Glu Thr Val Gln
    130                 135                 140

gag ttc aag caa gcg gct gcc cgc gca aaa gaa gcc ggc ttt gat gtg 480
Glu Phe Lys Gln Ala Ala Ala Arg Ala Lys Glu Ala Gly Phe Asp Val
145                 150                 155                 160

att gaa att cat gcg gcg cac gga tat tta att cat gaa ttt ttg tct 528
Ile Glu Ile His Ala Ala His Gly Tyr Leu Ile His Glu Phe Leu Ser
                165                 170                 175

ccg ctt tcc aac cat cga aca gat gaa tat ggc ggc tca cct gaa aac 576
Pro Leu Ser Asn His Arg Thr Asp Glu Tyr Gly Gly Ser Pro Glu Asn
            180                 185                 190

cgc tat cgt ttc ttg aga gag atc att gat gaa gtc aaa caa gta tgg 624
Arg Tyr Arg Phe Leu Arg Glu Ile Ile Asp Glu Val Lys Gln Val Trp
        195                 200                 205

gac ggt cct tta ttt gtc cgt gta tct gct tct gac tac act gat aaa 672
Asp Gly Pro Leu Phe Val Arg Val Ser Ala Ser Asp Tyr Thr Asp Lys
    210                 215                 220

ggc tta gac att gcc gat cac atc ggt ttt gca aaa tgg atg aag gag 720
Gly Leu Asp Ile Ala Asp His Ile Gly Phe Ala Lys Trp Met Lys Glu
225                 230                 235                 240

cag ggt gtt gac tta att gac tgc agc tca ggc gcc ctt gtt cac gca 768
Gln Gly Val Asp Leu Ile Asp Cys Ser Ser Gly Ala Leu Val His Ala
                245                 250                 255

gac att aac gta ttc cct ggc tat cag gtc agc ttc gct gag aaa atc 816
Asp Ile Asn Val Phe Pro Gly Tyr Gln Val Ser Phe Ala Glu Lys Ile
            260                 265                 270

cgt gaa cag gcg gac atg gct act ggt gcc gtc ggc atg att aca gac 864
Arg Glu Gln Ala Asp Met Ala Thr Gly Ala Val Gly Met Ile Thr Asp
        275                 280                 285

ggt tca atg gct gaa gaa att ctg caa aac gga cgt gcc gac ctc atc 912
Gly Ser Met Ala Glu Glu Ile Leu Gln Asn Gly Arg Ala Asp Leu Ile
    290                 295                 300

ttt atc ggc aga gag ctt ttg cgg gat cca ttt ttt gca aga act gct 960

Phe Ile Gly Arg Glu Leu Leu Arg Asp Pro Phe Phe Ala Arg Thr Ala
305                 310                 315                 320

gcg aaa cag ctc aat aca gag att ccg gcc cct gtt caa tac gaa aga       1008
Ala Lys Gln Leu Asn Thr Glu Ile Pro Ala Pro Val Gln Tyr Glu Arg
            325                 330                 335

ggc tgg taa                                                           1017
Gly Trp

<210>  14
<211>  338
<212>  PRT
<213>  Bacillus subtilis

<400>  14

Met Ala Arg Lys Leu Phe Thr Pro Ile Thr Ile Lys Asp Met Thr Leu
1               5                   10                  15

Lys Asn Arg Ile Val Met Ser Pro Met Cys Met Tyr Ser Ser His Glu
            20                  25                  30

Lys Asp Gly Lys Leu Thr Pro Phe His Met Ala His Tyr Ile Ser Arg
        35                  40                  45

Ala Ile Gly Gln Val Gly Leu Ile Ile Val Glu Ala Ser Ala Val Asn
    50                  55                  60

Pro Gln Gly Arg Ile Thr Asp Gln Asp Leu Gly Ile Trp Ser Asp Glu
65                  70                  75                  80

His Ile Glu Gly Phe Ala Lys Leu Thr Glu Gln Val Lys Glu Gln Gly
                85                  90                  95

Ser Lys Ile Gly Ile Gln Leu Ala His Ala Gly Arg Lys Ala Glu Leu
            100                 105                 110

Glu Gly Asp Ile Phe Ala Pro Ser Ala Ile Ala Phe Asp Glu Gln Ser
        115                 120                 125

Ala Thr Pro Val Glu Met Ser Ala Glu Lys Val Lys Glu Thr Val Gln
    130                 135                 140

Glu Phe Lys Gln Ala Ala Ala Arg Ala Lys Glu Ala Gly Phe Asp Val
145                 150                 155                 160

Ile Glu Ile His Ala Ala His Gly Tyr Leu Ile His Glu Phe Leu Ser
                165                 170                 175

Pro Leu Ser Asn His Arg Thr Asp Glu Tyr Gly Gly Ser Pro Glu Asn
            180                 185                 190

Arg Tyr Arg Phe Leu Arg Glu Ile Ile Asp Glu Val Lys Gln Val Trp
        195                 200                 205

Asp Gly Pro Leu Phe Val Arg Val Ser Ala Ser Asp Tyr Thr Asp Lys
    210                 215                 220

Gly Leu Asp Ile Ala Asp His Ile Gly Phe Ala Lys Trp Met Lys Glu
225                 230                 235                 240

Gln Gly Val Asp Leu Ile Asp Cys Ser Ser Gly Ala Leu Val His Ala
                245                 250                 255

Asp Ile Asn Val Phe Pro Gly Tyr Gln Val Ser Phe Ala Glu Lys Ile
            260                 265                 270

Arg Glu Gln Ala Asp Met Ala Thr Gly Ala Val Gly Met Ile Thr Asp
        275                 280                 285

Gly Ser Met Ala Glu Glu Ile Leu Gln Asn Gly Arg Ala Asp Leu Ile
    290                 295                 300

Phe Ile Gly Arg Glu Leu Leu Arg Asp Pro Phe Phe Ala Arg Thr Ala
305                 310                 315                 320

Ala Lys Gln Leu Asn Thr Glu Ile Pro Ala Pro Val Gln Tyr Glu Arg
                325                 330                 335

Gly Trp

<210> 15
<211> 1212
<212> DNA
<213> Artificial

<220>
<223> codon optimised OYE gene from Candida macedoniensis

<400> 15
atgtcctaca tgaactttga cccgaaaccg ctgggggata ccaacatctt caaaccgatt 60

aaaatcggta ataacgaact gaagcaccgt gttgtaatgc cggcgctgac gcgtatgcgt 120

gcaattgccc cgggtaacat tccgaacacc gaatgggctg aagaatacta tcgccagcgt 180

tctcagtacc cgggcactct gatcatcacc gaaggcacct tcccgtccgc gcagtctggt 240

ggttatccga atgttccggg tatttggtct aaggaacagc tggctgaatg gaagaaaatc 300

ttcaatgcga ttcacgaaaa caaaagcttc gtttgggttc aactgtgggt gctgggccgt 360

caggcttggc cggaagttct gaagaaagaa gggctgcgtt atgactccgc gaccgatgac 420

ctgtatatgg gtgaggaaga aaaagaacgc gcactgaaag caaacaaccc acaacacggg 480

attaccaagg aagaaattaa acagtacatc aaagagtatg ttgatgcggc gaaaaaggct 540

atcgacgccg gtgctgacgg cgtgcagatc cattccgcca acggttacct gctgaaccag 600

ttcctggacc cgatctccaa taaccgtacc gatgaatacg gtggctctat cgaaaatcgc 660

gctcgtttca ccctggaagt agttgacgcc gttgttgacg ccgtgggcgc ggagcgtacc 720

tccatccgct tctctccgta cggaacgttc ggtactatgt ccggaggcga gaacccggga 780

atcgtggctc aatacgctta cgttattggt gaactggaga aacgcgcacg cgctggtaaa 840

cgcctggcgt tcatcgatct ggtggaaccg cgcgttactg atccgttcct gccggagttt 900

gaaaaatggt ttaaagaagg taccaacgag ttcatctaca gcatttggaa aggtccggta 960

ctgcgtgttg gcaattatgc cctggacccg gaccaggcga ctctggatag caaaaagccg 1020

aacacgctga ttggctatgg tcgcagcttc atcgccaatc cagacctggt gtaccgcctg 1080

gaaaaaggac tgcctctgaa caaatatgac cgcaacactt tctacacttt cactaaggaa 1140

ggatacactg actacccgtc ctacgaggaa tctgtagcaa aagggtataa aaaggaagaa 1200

aagaaatact aa 1212

<210> 16
<211> 1197
<212> DNA
<213> Artificial

<220>
<223> codon optimised KYE1 gene from Kluyveromyces lactis NRRL Y-1140

<400> 16
atgagcttta tgaatttcga gcctaaaccg ctggccgata ctgatatctt caagccgatc 60

aaaattggta ataccgagct gaaacaccgt gtagttatgc cggctctgac tcgtatgcgc 120

gcgctgcatc caggtaacgt gccgaacccg gactgggctg ttgaatacta ccgtcagcgt 180

tcccaatatc ctggcaccat gatcatcact gaaggtgctt ttccgtctgc ccagtctggt 240

ggctacgata atgctcctgg cgtttggtct gaggaacaac tggcccagtg gcgcaagatc 300

tttaaagcga tccatgacaa caaaagcttc gtctgggtac agctgtgggt tctgggtcgt 360

caggcgttcg cagataacct ggctcgcgac ggtctgcgtt acgattctgc ctccgacgaa 420

gtttacatgg gtgaagatga gaaagaacgc gccatccgtt ccaacaaccc gcaacacggt 480

atcacgaaag atgaaatcaa gcagtacatc cgtgactacg tggacgcagc taaaaagtgt 540

atcgacgcgg gcgctgatgg tgttgaaatt cactctgcaa acggctacct gctgaaccag 600

tttctggacc ctatctccaa caaacgtact gacgaatatg gcggttctat cgagaatcgt 660

gcccgcttcg tactggaagt agttgacgcg gttgtggacg cggtgggcgc ggagcgtacc 720

agcattcgtt tcagcccata tggcgtattc ggtactatgt ccggtgtttc cgacccggtt 780

ctggttgcac agttcgcata tgtcctggcg gaactggaaa aacgtgcaaa agctggtaaa 840

cgtctggcat acgttgacct ggtggaaccg cgtgtaacct ccccattcca gccggaattt 900

gaaggctggt ataaaggcgg caccaacgaa ttcgtataca gcgtttggaa aggtaacgtt 960

ctgcgcgttg gcaactacgc tctggatccg gacgctgcaa ttaccgactc taaaaacccg 1020

aataccctga tcggctacgg ccgtgctttc atcgctaacc ctgatctggt agaacgtctg 1080

gagaaaggcc tgcctctgaa ccagtacgat cgtccttcct tttacaaaat gtccgccgag 1140

ggttatatcg attatccgac ttatgaagaa gcggttgcta agggctataa aaagtaa 1197

<210> 17
<211> 1050
<212> DNA
<213> Artificial

<220>
<223> codon optimised xenB gene from Pseudomonas fluorescens I-C

<400> 17
atggcgacta tttttgaccc gattaaactg ggagatctgg aactgtctaa ccgcattatc 60

atggcgccgc tgacccgttg ccgtgcggat gagggccgcg ttccaaatgc tctgatggct 120

gagtactatg tgcagcgtgc gtccgcaggt ctgattctgt ctgaagcgac ctccgtgact 180

cctatggggg taggctaccc ggatacgccg ggtatttggt ccaatgacca ggtgcgtgga 240

tggacgaaca tcaccaaagc tgttcacgca gcaggcggta agatcgttct gcaactgtgg 300

cacgttggcc gtatttccca cccgctgtac ctgaacggag aggcgccggt agctccgtct 360

gctatccaac cgaaaggtca tgtatccctg gtgcgtccgc tggctgatta tcctactccg 420

cgtgcgctgg aaaccgcgga gattgcggaa atcgttgaag catatcgtac gggagccgaa 480

aacgcgaaag ctgcgggctt tgacggcgtc gaaatccatg gcgctaacgg ttatctgctg 540

gatcagtttc tgcaaagctc cacgaatcag cgtaccgaca actacggcgg atctctggaa 600

aaccgtgcgc gcctgctgct ggaagtaacc gacgccgcaa ttgatgtgtg gggagccggc 660

cgtgttggtg ttcacctggc tcctcgtgcg gatagccacg acatgggtga cgacaacctg 720

gcagaaacct tcacttatgt ggctcgcgaa ctgggaaaac gcgggatcgc attcatctgt 780

tctcgcgaaa aagaaggtgc cgactccctg ggcccgcaac tgaaggaagc ctttggaggc 840

gcttatattg cgaacgagcg ttttaccaaa gattctgcaa acgcgtggct ggcggaaggt 900

aaagcagatg cagttgcgtt tggcgttccg ttcattgcca accctgacct gccggctcgc 960

ctgaaggcag acgctcctct gaacgaacct cgcccggaac tgttctacgg caaaggtccg 1020

gtgggatata tcgattaccc gaccctgtaa 1050

<210> 18
<211> 1083
<212> DNA
<213> Artificial

<220>
<223> codon optimised ncr gene from Pseudomonas syringae pv. glycinea

<400> 18
atgccaactc tgtttgaccc gctgactctg ggcgacctgc aatccccgaa ccgtgttctg 60

atggctcctc tgacccgtgg acgtgccacc cgtgagcacg tgccgaccga gctgatgatc 120

gaatactata cccagcgcgc gagcgcaggc ctgatcatca ccgaagccac gggcatcacc 180

caggaaggtc tgggctggcc gtatgctccg gggatctggt ccgacgagca ggtggaagcc 240

tggaaaccgg tgactcaggc tgtacatgaa gcgggtggac gcattatcct gcaactgtgg 300

cacatgggtc gcaccgttca ttcttctttc ctgggtggcg caaaacctgt ttctagctcc 360

gcaacccgtg caccgggaca ggctcacact tacgaaggga aacaggatta cgacgaagcg 420

cgtccgctga gcgccgatga aattccgcgt ctgctgaacg attatgagca cgctgccaaa 480

aacgcaatgg cagcaggctt cgatggtgtc cagattcacg ccgctaacgg ctatctgatc 540

gaccaatttc tgcgtgataa ctccaacgtt cgcggggacg catacggtgg ctctatcgag 600

aaccgtattc gcctgctggt tgaagttacc cgtcgtgttg ccgaaaccgt gggagccgag 660

aaaaccggtg tgcgtctgtc cccgaatggc gactcccagg gggtgaacga ttctaaccca 720

gaaccgctgt tttctgcggc ggctaaagca ctggatgaga ttggtatcgc ccacctggaa 780

ctgcgtgaac cgggctatga aggcactttt ggcaaagctg accgtccgcc ggtacatcca 840

gtcatccgtc aggcattttc tcgtaccctg atcctgaaca gcgattacac gctggaaacc 900

gcacaggctg cactggcaac cggtgaagct gatgctatta cttttggccg tccgtttctg 960

gcgaaccctg acctgccgca tcgtttcgcg gaacgtctgc cgctgaacaa ggatgttatg 1020

gaaacctggt actctcaggg accggagggt tacgtagatt atccaaccgc agatcagaaa 1080

taa 1083

SEQUENCE LISTING

<110>  DSM IP ASSETS B.V.

<120>  Preparation of (Z)-6,7-dihydro-1H-azepin-2(5H)-one

<130>  P84471EP00

<160>  18

<170>  PatentIn version 3.5

<210>  1
<211>  1212
<212>  DNA
<213>  Candida macedoniensis


<220>
<221>  CDS
<222>  (1)..(1212)

<400>  1
atg tcg tac atg aac ttt gac cct aag cca ttg gga gac acc aat atc      48
Met Ser Tyr Met Asn Phe Asp Pro Lys Pro Leu Gly Asp Thr Asn Ile
1               5                   10                  15

ttc aag cca atc aag atc ggt aac aat gag cta aaa cac aga gta gtc      96
Phe Lys Pro Ile Lys Ile Gly Asn Asn Glu Leu Lys His Arg Val Val
            20                  25                  30

atg cca gca ttg act aga atg aga gcc att gca cca gga aac atc cca      144
Met Pro Ala Leu Thr Arg Met Arg Ala Ile Ala Pro Gly Asn Ile Pro
        35                  40                  45

aac act gaa tgg gcc gag gaa tac tac aga caa cgt tct caa tac cct      192
Asn Thr Glu Trp Ala Glu Glu Tyr Tyr Arg Gln Arg Ser Gln Tyr Pro
    50                  55                  60

ggt acc ctt att atc acg gaa ggt act ttc cct tct gcg caa tca ggt      240
Gly Thr Leu Ile Ile Thr Glu Gly Thr Phe Pro Ser Ala Gln Ser Gly
65                  70                  75                  80

ggt tac cca aat gtg cca ggt atc tgg tcc aaa gag caa ttg gct gaa      288
Gly Tyr Pro Asn Val Pro Gly Ile Trp Ser Lys Glu Gln Leu Ala Glu
                85                  90                  95

tgg aaa aag atc ttc aat gca atc cat gag aac aaa tcg ttc gtg tgg      336
Trp Lys Lys Ile Phe Asn Ala Ile His Glu Asn Lys Ser Phe Val Trp
            100                 105                 110

gtg caa ttg tgg gtt cta ggt aga caa gca tgg cca gaa gtg ttg aag      384
Val Gln Leu Trp Val Leu Gly Arg Gln Ala Trp Pro Glu Val Leu Lys
        115                 120                 125

aag gaa ggt ttg cgt tac gat agt gct acc gat gac ttg tac atg ggt      432
Lys Glu Gly Leu Arg Tyr Asp Ser Ala Thr Asp Asp Leu Tyr Met Gly
    130                 135                 140

gaa gaa gaa aaa gag cgt gcc tta aag gct aac aac cca cag cac ggt      480
Glu Glu Glu Lys Glu Arg Ala Leu Lys Ala Asn Asn Pro Gln His Gly
145                 150                 155                 160

atc acc aag gaa gaa atc aag cag tac atc aag gag tac gtg gat gct      528
Ile Thr Lys Glu Glu Ile Lys Gln Tyr Ile Lys Glu Tyr Val Asp Ala
                165                 170                 175

gcc aag aaa gcc atc gat gca ggt gca gac ggt gtg caa atc cat tct      576

Ala Lys Lys Ala Ile Asp Ala Gly Ala Asp Gly Val Gln Ile His Ser
            180                 185                 190

gcc aac ggt tac ttg ttg aac cag ttt ttg gac cct att tct aac aac      624
Ala Asn Gly Tyr Leu Leu Asn Gln Phe Leu Asp Pro Ile Ser Asn Asn
        195                 200                 205

aga acc gac gag tac ggt gga tcg atc gag aac cgt gcg aga ttc act      672
Arg Thr Asp Glu Tyr Gly Gly Ser Ile Glu Asn Arg Ala Arg Phe Thr
    210                 215                 220

ttg gaa gtg gtc gat gcc gtt gtc gat gca gtt ggt gcc gaa aga acc      720
Leu Glu Val Val Asp Ala Val Val Asp Ala Val Gly Ala Glu Arg Thr
225                 230                 235                 240

tcc atc aga ttc tct cca tac ggt act ttt ggt acc atg tcc ggt ggt      768
Ser Ile Arg Phe Ser Pro Tyr Gly Thr Phe Gly Thr Met Ser Gly Gly
                245                 250                 255

gag aac cct ggc atc gtt gct caa tat gca tac gtc att ggt gag ttg      816
Glu Asn Pro Gly Ile Val Ala Gln Tyr Ala Tyr Val Ile Gly Glu Leu
            260                 265                 270

gaa aag aga gct aga gct ggc aag aga ttg gcg ttc atc gat ttg gtc      864
Glu Lys Arg Ala Arg Ala Gly Lys Arg Leu Ala Phe Ile Asp Leu Val
        275                 280                 285

gag cct cgt gtg acc gac cca ttc cta cca gaa ttc gag aag tgg ttc      912
Glu Pro Arg Val Thr Asp Pro Phe Leu Pro Glu Phe Glu Lys Trp Phe
    290                 295                 300

aag gaa ggt acc aac gaa ttc atc tac tct atc tgg aag ggt cca gtt      960
Lys Glu Gly Thr Asn Glu Phe Ile Tyr Ser Ile Trp Lys Gly Pro Val
305                 310                 315                 320

ctc aga gtt ggt aac tat gct ttg gac cca gat caa gcc act ctc gac     1008
Leu Arg Val Gly Asn Tyr Ala Leu Asp Pro Asp Gln Ala Thr Leu Asp
                325                 330                 335

tct aag aag cct aac act ttg atc ggt tac ggt aga tcc ttc atc gcc     1056
Ser Lys Lys Pro Asn Thr Leu Ile Gly Tyr Gly Arg Ser Phe Ile Ala
            340                 345                 350

aac cca gac ttg gtg tac cgt ttg gaa aag ggt ttg cca ttg aac aag     1104
Asn Pro Asp Leu Val Tyr Arg Leu Glu Lys Gly Leu Pro Leu Asn Lys
        355                 360                 365

tat gat aga aac acc ttt tac aca ttc act aag gaa ggt tac acc gat     1152
Tyr Asp Arg Asn Thr Phe Tyr Thr Phe Thr Lys Glu Gly Tyr Thr Asp
    370                 375                 380

tac cca agc tac gaa gaa tcc gtc gca aag ggt tac aag aaa gag gaa     1200
Tyr Pro Ser Tyr Glu Glu Ser Val Ala Lys Gly Tyr Lys Lys Glu Glu
385                 390                 395                 400

aag aag tac taa                                                     1212
Lys Lys Tyr

<210>  2
<211>  403
<212>  PRT
<213>  Candida macedoniensis

<400>  2

Met Ser Tyr Met Asn Phe Asp Pro Lys Pro Leu Gly Asp Thr Asn Ile

1                   5                   10                  15

Phe Lys Pro Ile Lys Ile Gly Asn Asn Glu Leu Lys His Arg Val Val
            20                  25                  30

Met Pro Ala Leu Thr Arg Met Arg Ala Ile Ala Pro Gly Asn Ile Pro
        35                  40                  45

Asn Thr Glu Trp Ala Glu Glu Tyr Tyr Arg Gln Arg Ser Gln Tyr Pro
    50                  55                  60

Gly Thr Leu Ile Ile Thr Glu Gly Thr Phe Pro Ser Ala Gln Ser Gly
65                  70                  75                  80

Gly Tyr Pro Asn Val Pro Gly Ile Trp Ser Lys Glu Gln Leu Ala Glu
                85                  90                  95

Trp Lys Lys Ile Phe Asn Ala Ile His Glu Asn Lys Ser Phe Val Trp
            100                 105                 110

Val Gln Leu Trp Val Leu Gly Arg Gln Ala Trp Pro Glu Val Leu Lys
        115                 120                 125

Lys Glu Gly Leu Arg Tyr Asp Ser Ala Thr Asp Asp Leu Tyr Met Gly
    130                 135                 140

Glu Glu Glu Lys Glu Arg Ala Leu Lys Ala Asn Asn Pro Gln His Gly
145                 150                 155                 160

Ile Thr Lys Glu Glu Ile Lys Gln Tyr Ile Lys Glu Tyr Val Asp Ala
                165                 170                 175

Ala Lys Lys Ala Ile Asp Ala Gly Ala Asp Gly Val Gln Ile His Ser
            180                 185                 190

Ala Asn Gly Tyr Leu Leu Asn Gln Phe Leu Asp Pro Ile Ser Asn Asn
        195                 200                 205

Arg Thr Asp Glu Tyr Gly Gly Ser Ile Glu Asn Arg Ala Arg Phe Thr
    210                 215                 220

Leu Glu Val Val Asp Ala Val Val Asp Ala Val Gly Ala Glu Arg Thr
225                 230                 235                 240

Ser Ile Arg Phe Ser Pro Tyr Gly Thr Phe Gly Thr Met Ser Gly Gly
                245                 250                 255

Glu Asn Pro Gly Ile Val Ala Gln Tyr Ala Tyr Val Ile Gly Glu Leu
            260                 265                 270

Glu Lys Arg Ala Arg Ala Gly Lys Arg Leu Ala Phe Ile Asp Leu Val

275                 280                 285

Glu Pro Arg Val Thr Asp Pro Phe Leu Pro Glu Phe Glu Lys Trp Phe
    290                 295                 300

Lys Glu Gly Thr Asn Glu Phe Ile Tyr Ser Ile Trp Lys Gly Pro Val
305                 310                 315                 320

Leu Arg Val Gly Asn Tyr Ala Leu Asp Pro Asp Gln Ala Thr Leu Asp
                325                 330                 335

Ser Lys Lys Pro Asn Thr Leu Ile Gly Tyr Gly Arg Ser Phe Ile Ala
            340                 345                 350

Asn Pro Asp Leu Val Tyr Arg Leu Glu Lys Gly Leu Pro Leu Asn Lys
        355                 360                 365

Tyr Asp Arg Asn Thr Phe Tyr Thr Phe Thr Lys Glu Gly Tyr Thr Asp
    370                 375                 380

Tyr Pro Ser Tyr Glu Glu Ser Val Ala Lys Gly Tyr Lys Lys Glu Glu
385                 390                 395                 400

Lys Lys Tyr

<210>  3
<211>  1197
<212>  DNA
<213>  Kluyveromyces lactis

<220>
<221>  CDS
<222>  (1)..(1197)

<400>  3
atg tcg ttt atg aac ttt gaa cca aag cca ttg gct gat act gat atc      48
Met Ser Phe Met Asn Phe Glu Pro Lys Pro Leu Ala Asp Thr Asp Ile
1               5                   10                  15

ttc aaa cca atc aag att ggt aac act gaa ttg aag cac agg gtt gtc      96
Phe Lys Pro Ile Lys Ile Gly Asn Thr Glu Leu Lys His Arg Val Val
            20                  25                  30

atg cct gca ttg aca aga atg aga gcg ttg cat cca ggc aac gtt cca      144
Met Pro Ala Leu Thr Arg Met Arg Ala Leu His Pro Gly Asn Val Pro
        35                  40                  45

aac cct gac tgg gct gtt gaa tat tac aga caa cgt tcc caa tat cca      192
Asn Pro Asp Trp Ala Val Glu Tyr Tyr Arg Gln Arg Ser Gln Tyr Pro
    50                  55                  60

ggt act atg att atc act gaa ggt gct ttc cca tca gct cag tca ggt      240
Gly Thr Met Ile Ile Thr Glu Gly Ala Phe Pro Ser Ala Gln Ser Gly
65                  70                  75                  80

ggt tac gat aac gca cca ggt gtt tgg agc gaa gaa caa ctg gct caa      288
Gly Tyr Asp Asn Ala Pro Gly Val Trp Ser Glu Glu Gln Leu Ala Gln

                85                  90                  95

tgg aga aag atc ttc aag gca att cac gac aac aag tct ttt gtt tgg      336
Trp Arg Lys Ile Phe Lys Ala Ile His Asp Asn Lys Ser Phe Val Trp
            100                 105                 110

gta caa ttg tgg gtt cta ggt aga caa gct ttt gct gat aac ttg gca      384
Val Gln Leu Trp Val Leu Gly Arg Gln Ala Phe Ala Asp Asn Leu Ala
        115                 120                 125

aga gat gga ttg cgt tat gat agt gct tcc gat gaa gtg tac atg ggt      432
Arg Asp Gly Leu Arg Tyr Asp Ser Ala Ser Asp Glu Val Tyr Met Gly
    130                 135                 140

gaa gat gaa aag gaa cgt gcc atc aga tct aac aac cct cag cat ggt      480
Glu Asp Glu Lys Glu Arg Ala Ile Arg Ser Asn Asn Pro Gln His Gly
145                 150                 155                 160

atc acc aag gat gaa att aag cag tat atc agg gac tat gtt gat gct      528
Ile Thr Lys Asp Glu Ile Lys Gln Tyr Ile Arg Asp Tyr Val Asp Ala
                165                 170                 175

gct aag aag tgt atc gat gct ggt gca gat ggt gtt gaa atc cat tcc      576
Ala Lys Lys Cys Ile Asp Ala Gly Ala Asp Gly Val Glu Ile His Ser
            180                 185                 190

gct aac ggt tat ttg ttg aat caa ttc cta gac cca atc tcc aac aaa      624
Ala Asn Gly Tyr Leu Leu Asn Gln Phe Leu Asp Pro Ile Ser Asn Lys
        195                 200                 205

aga act gat gaa tac ggt gga tcc att gag aac cgt gct aga ttc gtc      672
Arg Thr Asp Glu Tyr Gly Gly Ser Ile Glu Asn Arg Ala Arg Phe Val
    210                 215                 220

ttg gaa gtc gtc gat gcc gtt gtc gat gcc gtt ggt gcc gaa aga acc      720
Leu Glu Val Val Asp Ala Val Val Asp Ala Val Gly Ala Glu Arg Thr
225                 230                 235                 240

agt atc aga ttc tca cca tac ggt gta ttt ggt acc atg tca ggt gtt      768
Ser Ile Arg Phe Ser Pro Tyr Gly Val Phe Gly Thr Met Ser Gly Val
                245                 250                 255

tca gac cct gtc ttg gtg gct caa ttc gcc tat gta ctt gct gaa ttg      816
Ser Asp Pro Val Leu Val Ala Gln Phe Ala Tyr Val Leu Ala Glu Leu
            260                 265                 270

gaa aag agg gca aag gct ggt aag aga tta gca tac gtc gat tta gtc      864
Glu Lys Arg Ala Lys Ala Gly Lys Arg Leu Ala Tyr Val Asp Leu Val
        275                 280                 285

gaa cct cgt gtc aca tcg cca ttc caa ccg gaa ttt gaa ggc tgg tat      912
Glu Pro Arg Val Thr Ser Pro Phe Gln Pro Glu Phe Glu Gly Trp Tyr
    290                 295                 300

aaa ggt ggt acc aat gaa ttc gta tac tct gtt tgg aag ggt aac gtg      960
Lys Gly Gly Thr Asn Glu Phe Val Tyr Ser Val Trp Lys Gly Asn Val
305                 310                 315                 320

cta aga gtt ggt aac tac gct ttg gac cca gat gct gcc att acg gac     1008
Leu Arg Val Gly Asn Tyr Ala Leu Asp Pro Asp Ala Ala Ile Thr Asp
                325                 330                 335

tca aag aat cca aac act ttg atc ggt tac ggt aga gcc ttc att gcc     1056
Ser Lys Asn Pro Asn Thr Leu Ile Gly Tyr Gly Arg Ala Phe Ile Ala
            340                 345                 350

aac cca gat ctt gtt gaa cgt ctc gaa aag ggt ttg cca ttg aat caa     1104
Asn Pro Asp Leu Val Glu Arg Leu Glu Lys Gly Leu Pro Leu Asn Gln

        355                 360                 365

tac gat aga ccc tct ttc tac aaa atg tct gcg gaa ggg tat atc gac 1152
Tyr Asp Arg Pro Ser Phe Tyr Lys Met Ser Ala Glu Gly Tyr Ile Asp
    370                 375                 380

tac cca aca tac gag gaa gct gtt gcc aag ggt tac aag aaa tag 1197
Tyr Pro Thr Tyr Glu Glu Ala Val Ala Lys Gly Tyr Lys Lys
385                 390                 395

<210> 4
<211> 398
<212> PRT
<213> Kluyveromyces lactis

<400> 4

Met Ser Phe Met Asn Phe Glu Pro Lys Pro Leu Ala Asp Thr Asp Ile
1               5                   10                  15

Phe Lys Pro Ile Lys Ile Gly Asn Thr Glu Leu Lys His Arg Val Val
            20                  25                  30

Met Pro Ala Leu Thr Arg Met Arg Ala Leu His Pro Gly Asn Val Pro
        35                  40                  45

Asn Pro Asp Trp Ala Val Glu Tyr Tyr Arg Gln Arg Ser Gln Tyr Pro
    50                  55                  60

Gly Thr Met Ile Ile Thr Glu Gly Ala Phe Pro Ser Ala Gln Ser Gly
65                  70                  75                  80

Gly Tyr Asp Asn Ala Pro Gly Val Trp Ser Glu Glu Gln Leu Ala Gln
                85                  90                  95

Trp Arg Lys Ile Phe Lys Ala Ile His Asp Asn Lys Ser Phe Val Trp
            100                 105                 110

Val Gln Leu Trp Val Leu Gly Arg Gln Ala Phe Ala Asp Asn Leu Ala
        115                 120                 125

Arg Asp Gly Leu Arg Tyr Asp Ser Ala Ser Asp Glu Val Tyr Met Gly
    130                 135                 140

Glu Asp Glu Lys Glu Arg Ala Ile Arg Ser Asn Asn Pro Gln His Gly
145                 150                 155                 160

Ile Thr Lys Asp Glu Ile Lys Gln Tyr Ile Arg Asp Tyr Val Asp Ala
                165                 170                 175

Ala Lys Lys Cys Ile Asp Ala Gly Ala Asp Gly Val Glu Ile His Ser
            180                 185                 190

Ala Asn Gly Tyr Leu Leu Asn Gln Phe Leu Asp Pro Ile Ser Asn Lys
        195                 200                 205

Arg Thr Asp Glu Tyr Gly Gly Ser Ile Glu Asn Arg Ala Arg Phe Val
    210                 215                 220

Leu Glu Val Val Asp Ala Val Val Asp Ala Val Gly Ala Glu Arg Thr
225                 230                 235                 240

Ser Ile Arg Phe Ser Pro Tyr Gly Val Phe Gly Thr Met Ser Gly Val
                245                 250                 255

Ser Asp Pro Val Leu Val Ala Gln Phe Ala Tyr Val Leu Ala Glu Leu
            260                 265                 270

Glu Lys Arg Ala Lys Ala Gly Lys Arg Leu Ala Tyr Val Asp Leu Val
        275                 280                 285

Glu Pro Arg Val Thr Ser Pro Phe Gln Pro Glu Phe Glu Gly Trp Tyr
    290                 295                 300

Lys Gly Gly Thr Asn Glu Phe Val Tyr Ser Val Trp Lys Gly Asn Val
305                 310                 315                 320

Leu Arg Val Gly Asn Tyr Ala Leu Asp Pro Asp Ala Ala Ile Thr Asp
                325                 330                 335

Ser Lys Asn Pro Asn Thr Leu Ile Gly Tyr Gly Arg Ala Phe Ile Ala
            340                 345                 350

Asn Pro Asp Leu Val Glu Arg Leu Glu Lys Gly Leu Pro Leu Asn Gln
        355                 360                 365

Tyr Asp Arg Pro Ser Phe Tyr Lys Met Ser Ala Glu Gly Tyr Ile Asp
    370                 375                 380

Tyr Pro Thr Tyr Glu Glu Ala Val Ala Lys Gly Tyr Lys Lys
385                 390                 395

<210>  5
<211>  1050
<212>  DNA
<213>  Pseudomonas fluorescens

<220>
<221>  CDS
<222>  (1)..(1050)

<400>  5
atg gca act att ttc gat ccg atc aaa ctg ggc gac ctc gag ctg tcc      48
Met Ala Thr Ile Phe Asp Pro Ile Lys Leu Gly Asp Leu Glu Leu Ser
1               5                   10                  15

aac cgc atc atc atg gcc ccg ctg act cgc tgc cgc gcc gac gaa ggc      96
Asn Arg Ile Ile Met Ala Pro Leu Thr Arg Cys Arg Ala Asp Glu Gly
            20                  25                  30

cgc gta ccc aac gca ctg atg gcc gag tac tac gtg caa cgt gcc tcc 144
Arg Val Pro Asn Ala Leu Met Ala Glu Tyr Tyr Val Gln Arg Ala Ser
        35                  40                  45

gcc ggc ctg att ctc agc gaa gcc act tcg gtg acg ccg atg ggc gtc 192
Ala Gly Leu Ile Leu Ser Glu Ala Thr Ser Val Thr Pro Met Gly Val
    50                  55                  60

ggc tat ccg gac acc ccg ggc atc tgg tcc aac gat cag gta cgc ggc 240
Gly Tyr Pro Asp Thr Pro Gly Ile Trp Ser Asn Asp Gln Val Arg Gly
65                  70                  75                  80

tgg acc aac atc acc aaa gcc gta cac gct gcc ggc ggc aag atc gtc 288
Trp Thr Asn Ile Thr Lys Ala Val His Ala Ala Gly Gly Lys Ile Val
                85                  90                  95

ctg caa ctt tgg cac gtc ggc cgc atc tcg cac ccg ttg tac ctg aac 336
Leu Gln Leu Trp His Val Gly Arg Ile Ser His Pro Leu Tyr Leu Asn
            100                 105                 110

ggc gaa gca ccg gtc gcg ccg agc gcc atc cag cct aaa ggc cac gtc 384
Gly Glu Ala Pro Val Ala Pro Ser Ala Ile Gln Pro Lys Gly His Val
        115                 120                 125

agc ctg gtg cgt cca ctg gcc gat tac ccg act cca cgc gcc ctg gaa 432
Ser Leu Val Arg Pro Leu Ala Asp Tyr Pro Thr Pro Arg Ala Leu Glu
    130                 135                 140

acc gct gaa atc gcc gag atc gtc gag gcc tac cgc acc ggt gcc gag 480
Thr Ala Glu Ile Ala Glu Ile Val Glu Ala Tyr Arg Thr Gly Ala Glu
145                 150                 155                 160

aac gcc aag gcc gcc ggt ttc gac ggc gtg gaa atc cac ggc gcc aac 528
Asn Ala Lys Ala Ala Gly Phe Asp Gly Val Glu Ile His Gly Ala Asn
                165                 170                 175

ggc tac ctg ctc gac cag ttc ttg caa agc agc acc aac cag cgc acc 576
Gly Tyr Leu Leu Asp Gln Phe Leu Gln Ser Ser Thr Asn Gln Arg Thr
            180                 185                 190

gac aat tac ggc ggc tcc ctg gaa aac cgt gcg cgt ctg ttg ctg gaa 624
Asp Asn Tyr Gly Gly Ser Leu Glu Asn Arg Ala Arg Leu Leu Leu Glu
        195                 200                 205

gtg act gat gcc gcg atc gac gtc tgg ggc gcc ggc cgt gtc ggt gtg 672
Val Thr Asp Ala Ala Ile Asp Val Trp Gly Ala Gly Arg Val Gly Val
    210                 215                 220

cac ctg gca ccg cgc gcc gac tcc cac gac atg ggc gac gac aac ctc 720
His Leu Ala Pro Arg Ala Asp Ser His Asp Met Gly Asp Asp Asn Leu
225                 230                 235                 240

gcc gag acc ttc acc tat gtt gct cgc gag ctg ggc aag cgt ggc atc 768
Ala Glu Thr Phe Thr Tyr Val Ala Arg Glu Leu Gly Lys Arg Gly Ile
                245                 250                 255

gcc ttc atc tgc tcc cgc gag aaa gaa ggc gcc gac agc ctc ggc cca 816
Ala Phe Ile Cys Ser Arg Glu Lys Glu Gly Ala Asp Ser Leu Gly Pro
            260                 265                 270

caa ctg aaa gaa gcc ttt ggc ggc gcg tac atc gcc aac gag cgt ttc 864
Gln Leu Lys Glu Ala Phe Gly Gly Ala Tyr Ile Ala Asn Glu Arg Phe
        275                 280                 285

acc aag gac agc gcc aat gcg tgg ctg gct gaa ggc aag gct gac gct 912
Thr Lys Asp Ser Ala Asn Ala Trp Leu Ala Glu Gly Lys Ala Asp Ala
    290                 295                 300

gta gcg ttc ggc gtg cca ttc att gcc aac ccg gac ctg ccg gca cgc 960
Val Ala Phe Gly Val Pro Phe Ile Ala Asn Pro Asp Leu Pro Ala Arg
305                 310                 315                 320

ctg aaa gcc gat gcc ccg ctg aac gag ccg cgt cct gag ctg ttc tat 1008
Leu Lys Ala Asp Ala Pro Leu Asn Glu Pro Arg Pro Glu Leu Phe Tyr
                325                 330                 335

ggc aaa ggc ccg gtc ggc tac atc gac tac ccg acg ctg taa 1050
Gly Lys Gly Pro Val Gly Tyr Ile Asp Tyr Pro Thr Leu
            340                 345

<210> 6
<211> 349
<212> PRT
<213> Pseudomonas fluorescens

<400> 6

Met Ala Thr Ile Phe Asp Pro Ile Lys Leu Gly Asp Leu Glu Leu Ser
1               5                   10                  15

Asn Arg Ile Ile Met Ala Pro Leu Thr Arg Cys Arg Ala Asp Glu Gly
            20                  25                  30

Arg Val Pro Asn Ala Leu Met Ala Glu Tyr Tyr Val Gln Arg Ala Ser
        35                  40                  45

Ala Gly Leu Ile Leu Ser Glu Ala Thr Ser Val Thr Pro Met Gly Val
    50                  55                  60

Gly Tyr Pro Asp Thr Pro Gly Ile Trp Ser Asn Asp Gln Val Arg Gly
65                  70                  75                  80

Trp Thr Asn Ile Thr Lys Ala Val His Ala Ala Gly Gly Lys Ile Val
                85                  90                  95

Leu Gln Leu Trp His Val Gly Arg Ile Ser His Pro Leu Tyr Leu Asn
            100                 105                 110

Gly Glu Ala Pro Val Ala Pro Ser Ala Ile Gln Pro Lys Gly His Val
        115                 120                 125

Ser Leu Val Arg Pro Leu Ala Asp Tyr Pro Thr Pro Arg Ala Leu Glu
    130                 135                 140

Thr Ala Glu Ile Ala Glu Ile Val Glu Ala Tyr Arg Thr Gly Ala Glu
145                 150                 155                 160

Asn Ala Lys Ala Ala Gly Phe Asp Gly Val Glu Ile His Gly Ala Asn
                165                 170                 175

Gly Tyr Leu Leu Asp Gln Phe Leu Gln Ser Ser Thr Asn Gln Arg Thr
            180                 185                 190

Asp Asn Tyr Gly Gly Ser Leu Glu Asn Arg Ala Arg Leu Leu Leu Glu
        195                 200                 205

Val Thr Asp Ala Ala Ile Asp Val Trp Gly Ala Gly Arg Val Gly Val
    210                 215                 220

His Leu Ala Pro Arg Ala Asp Ser His Asp Met Gly Asp Asp Asn Leu
225                 230                 235                 240

Ala Glu Thr Phe Thr Tyr Val Ala Arg Glu Leu Gly Lys Arg Gly Ile
                245                 250                 255

Ala Phe Ile Cys Ser Arg Glu Lys Glu Gly Ala Asp Ser Leu Gly Pro
            260                 265                 270

Gln Leu Lys Glu Ala Phe Gly Gly Ala Tyr Ile Ala Asn Glu Arg Phe
        275                 280                 285

Thr Lys Asp Ser Ala Asn Ala Trp Leu Ala Glu Gly Lys Ala Asp Ala
    290                 295                 300

Val Ala Phe Gly Val Pro Phe Ile Ala Asn Pro Asp Leu Pro Ala Arg
305                 310                 315                 320

Leu Lys Ala Asp Ala Pro Leu Asn Glu Pro Arg Pro Glu Leu Phe Tyr
                325                 330                 335

Gly Lys Gly Pro Val Gly Tyr Ile Asp Tyr Pro Thr Leu
            340                 345

<210>  7
<211>  1083
<212>  DNA
<213>  Pseudomonas syringae

<220>
<221>  CDS
<222>  (1)..(1083)

<400>  7
atg ccg act ctt ttc gac ccc ttg act ttg ggc gac ctg caa tct cca      48
Met Pro Thr Leu Phe Asp Pro Leu Thr Leu Gly Asp Leu Gln Ser Pro
1               5                   10                  15

aac cgt gtt ctg atg gca ccg cta acg cgt ggc cgc gcg acc cgc gag      96
Asn Arg Val Leu Met Ala Pro Leu Thr Arg Gly Arg Ala Thr Arg Glu
            20                  25                  30

cac gtg cct acc gag ctg atg atc gag tat tac acc cag cgt gcc agc      144
His Val Pro Thr Glu Leu Met Ile Glu Tyr Tyr Thr Gln Arg Ala Ser
        35                  40                  45

gcg ggc ctg atc atc acc gaa gcc acc ggc atc acc caa gaa ggc cta      192
Ala Gly Leu Ile Ile Thr Glu Ala Thr Gly Ile Thr Gln Glu Gly Leu
    50                  55                  60

ggc tgg ccc tat gcg ccc ggc att tgg agc gat gaa cag gtc gag gcc 240
Gly Trp Pro Tyr Ala Pro Gly Ile Trp Ser Asp Glu Gln Val Glu Ala
65                  70                  75                  80

tgg aag ccg gtg acc cag gcc gtg cat gag gca ggc gga cgg atc att 288
Trp Lys Pro Val Thr Gln Ala Val His Glu Ala Gly Gly Arg Ile Ile
                85                  90                  95

ctt cag ttg tgg cat atg ggc cgt acc gtt cat tcc agc ttt ctc ggc 336
Leu Gln Leu Trp His Met Gly Arg Thr Val His Ser Ser Phe Leu Gly
            100                 105                 110

gga gcc aag cca gta tcg tcc tcg gcc acc cgt gcg ccg gga cag gcg 384
Gly Ala Lys Pro Val Ser Ser Ser Ala Thr Arg Ala Pro Gly Gln Ala
        115                 120                 125

cac acc tac gaa ggc aag caa gac tac gac gag gcg cgg cct ttg tcg 432
His Thr Tyr Glu Gly Lys Gln Asp Tyr Asp Glu Ala Arg Pro Leu Ser
    130                 135                 140

gcg gat gaa atc ccg cgg cta ttg aac gat tac gaa cac gca gcg aaa 480
Ala Asp Glu Ile Pro Arg Leu Leu Asn Asp Tyr Glu His Ala Ala Lys
145                 150                 155                 160

aac gcc atg gcc gca ggc ttc gac ggc gtg cag atc cat gct gcc aat 528
Asn Ala Met Ala Ala Gly Phe Asp Gly Val Gln Ile His Ala Ala Asn
                165                 170                 175

ggt tac cta atc gac cag ttc ctg cgc gac aac agc aac gtt cgc ggg 576
Gly Tyr Leu Ile Asp Gln Phe Leu Arg Asp Asn Ser Asn Val Arg Gly
            180                 185                 190

gac gcc tac ggg ggt tca atc gag aac cgc atc cgt cta ttg gtc gaa 624
Asp Ala Tyr Gly Gly Ser Ile Glu Asn Arg Ile Arg Leu Leu Val Glu
        195                 200                 205

gtc acc cgg cgc gtg gcg gag acc gta ggt gcc gaa aaa acg ggc gtg 672
Val Thr Arg Arg Val Ala Glu Thr Val Gly Ala Glu Lys Thr Gly Val
    210                 215                 220

cgg ctg tca ccc aac ggt gat tcc caa ggc gtc aac gac agc aat ccg 720
Arg Leu Ser Pro Asn Gly Asp Ser Gln Gly Val Asn Asp Ser Asn Pro
225                 230                 235                 240

gag ccg ctg ttc agc gcc gcg gcc aag gcc ttg gat gag atc ggc atc 768
Glu Pro Leu Phe Ser Ala Ala Ala Lys Ala Leu Asp Glu Ile Gly Ile
                245                 250                 255

gct cat ctg gag ttg cgc gaa cca ggg tat gaa ggc acc ttc ggc aag 816
Ala His Leu Glu Leu Arg Glu Pro Gly Tyr Glu Gly Thr Phe Gly Lys
            260                 265                 270

gcc gac cgg ccg ccc gtg cac ccg gtc atc cgc cag gcg ttc agc cgt 864
Ala Asp Arg Pro Pro Val His Pro Val Ile Arg Gln Ala Phe Ser Arg
        275                 280                 285

acg ctg att ctc aac tct gac tac act ttg gaa acg gct cag gct gca 912
Thr Leu Ile Leu Asn Ser Asp Tyr Thr Leu Glu Thr Ala Gln Ala Ala
    290                 295                 300

cta gcc acc gga gaa gcg gac gcg atc acc ttc ggc cgc ccg ttc ctg 960
Leu Ala Thr Gly Glu Ala Asp Ala Ile Thr Phe Gly Arg Pro Phe Leu
305                 310                 315                 320

gcc aac cct gac ctg cct cac agg ttt gcc gag aga ctg ccg ctg aac 1008
Ala Asn Pro Asp Leu Pro His Arg Phe Ala Glu Arg Leu Pro Leu Asn
                325                 330                 335

aag gac gtg atg gag act tgg tat agc cag ggg ccc gaa ggt tat gtg 1056
Lys Asp Val Met Glu Thr Trp Tyr Ser Gln Gly Pro Glu Gly Tyr Val
            340                 345                 350

gac tac ccc acc gct gac caa aag tag 1083
Asp Tyr Pro Thr Ala Asp Gln Lys
        355                 360

<210> 8
<211> 360
<212> PRT
<213> Pseudomonas syringae

<400> 8

Met Pro Thr Leu Phe Asp Pro Leu Thr Leu Gly Asp Leu Gln Ser Pro
1               5                   10                  15

Asn Arg Val Leu Met Ala Pro Leu Thr Arg Gly Arg Ala Thr Arg Glu
            20                  25                  30

His Val Pro Thr Glu Leu Met Ile Glu Tyr Tyr Thr Gln Arg Ala Ser
        35                  40                  45

Ala Gly Leu Ile Ile Thr Glu Ala Thr Gly Ile Thr Gln Glu Gly Leu
    50                  55                  60

Gly Trp Pro Tyr Ala Pro Gly Ile Trp Ser Asp Glu Gln Val Glu Ala
65                  70                  75                  80

Trp Lys Pro Val Thr Gln Ala Val His Glu Ala Gly Gly Arg Ile Ile
                85                  90                  95

Leu Gln Leu Trp His Met Gly Arg Thr Val His Ser Ser Phe Leu Gly
            100                 105                 110

Gly Ala Lys Pro Val Ser Ser Ser Ala Thr Arg Ala Pro Gly Gln Ala
        115                 120                 125

His Thr Tyr Glu Gly Lys Gln Asp Tyr Asp Glu Ala Arg Pro Leu Ser
    130                 135                 140

Ala Asp Glu Ile Pro Arg Leu Leu Asn Asp Tyr Glu His Ala Ala Lys
145                 150                 155                 160

Asn Ala Met Ala Ala Gly Phe Asp Gly Val Gln Ile His Ala Ala Asn
                165                 170                 175

Gly Tyr Leu Ile Asp Gln Phe Leu Arg Asp Asn Ser Asn Val Arg Gly
            180                 185                 190

Asp Ala Tyr Gly Gly Ser Ile Glu Asn Arg Ile Arg Leu Leu Val Glu
        195                 200                 205

Val Thr Arg Arg Val Ala Glu Thr Val Gly Ala Glu Lys Thr Gly Val
    210                 215                 220

Arg Leu Ser Pro Asn Gly Asp Ser Gln Gly Val Asn Asp Ser Asn Pro
225                 230                 235                 240

Glu Pro Leu Phe Ser Ala Ala Ala Lys Ala Leu Asp Glu Ile Gly Ile
                245                 250                 255

Ala His Leu Glu Leu Arg Glu Pro Gly Tyr Glu Gly Thr Phe Gly Lys
            260                 265                 270

Ala Asp Arg Pro Pro Val His Pro Val Ile Arg Gln Ala Phe Ser Arg
        275                 280                 285

Thr Leu Ile Leu Asn Ser Asp Tyr Thr Leu Glu Thr Ala Gln Ala Ala
    290                 295                 300

Leu Ala Thr Gly Glu Ala Asp Ala Ile Thr Phe Gly Arg Pro Phe Leu
305                 310                 315                 320

Ala Asn Pro Asp Leu Pro His Arg Phe Ala Glu Arg Leu Pro Leu Asn
                325                 330                 335

Lys Asp Val Met Glu Thr Trp Tyr Ser Gln Gly Pro Glu Gly Tyr Val
            340                 345                 350

Asp Tyr Pro Thr Ala Asp Gln Lys
        355                 360

<210>  9
<211>  1098
<212>  DNA
<213>  Escherichia coli

<220>
<221>  CDS
<222>  (1)..(1098)

<400>  9
atg tca tct gaa aaa ctg tat tcc cca ctg aaa gtg ggc gcg atc acg      48
Met Ser Ser Glu Lys Leu Tyr Ser Pro Leu Lys Val Gly Ala Ile Thr
1               5                   10                  15

gcg gca aac cgt att ttt atg gca ccg ctg acg cgt ctg cgc agt att      96
Ala Ala Asn Arg Ile Phe Met Ala Pro Leu Thr Arg Leu Arg Ser Ile
            20                  25                  30

gaa ccg ggt gac att cct acc ccg ttg atg gcg gaa tac tat cgc caa      144
Glu Pro Gly Asp Ile Pro Thr Pro Leu Met Ala Glu Tyr Tyr Arg Gln
        35                  40                  45

cgt gcc agt gcc ggt ttg att att agt gaa gcc acg caa att tct gcc      192
Arg Ala Ser Ala Gly Leu Ile Ile Ser Glu Ala Thr Gln Ile Ser Ala
    50                  55                  60

cag gca aaa gga tat gca ggt gcg cct ggc atc cat agt ccg gag caa      240

Gln Ala Lys Gly Tyr Ala Gly Ala Pro Gly Ile His Ser Pro Glu Gln
65                  70                  75                  80

att gcc gca tgg aaa aaa atc acc gct ggc gtt cat gct gaa aat ggt     288
Ile Ala Ala Trp Lys Lys Ile Thr Ala Gly Val His Ala Glu Asn Gly
                85                  90                  95

cat atg gcc gtg cag ctg tgg cac acc gga cgc att tct cac gcc agc     336
His Met Ala Val Gln Leu Trp His Thr Gly Arg Ile Ser His Ala Ser
            100                 105                 110

ctg caa cct ggc ggt cag gca ccg gta gcg cct tca gca ctt agc gcg     384
Leu Gln Pro Gly Gly Gln Ala Pro Val Ala Pro Ser Ala Leu Ser Ala
        115                 120                 125

gga aca cgt act tct ctg cgc gat gaa aat ggt cag gcg atc cgt gtt     432
Gly Thr Arg Thr Ser Leu Arg Asp Glu Asn Gly Gln Ala Ile Arg Val
    130                 135                 140

gaa aca tcc atg ccg cgt gcg ctt gaa ctg gaa gag att cca ggt atc     480
Glu Thr Ser Met Pro Arg Ala Leu Glu Leu Glu Glu Ile Pro Gly Ile
145                 150                 155                 160

gtc aat gat ttc cgt cag gcc att gct aac gcg cgt gaa gcc ggt ttt     528
Val Asn Asp Phe Arg Gln Ala Ile Ala Asn Ala Arg Glu Ala Gly Phe
                165                 170                 175

gat ctg gta gag ctc cac tct gct cac ggt tat ttg ctg cat cag ttc     576
Asp Leu Val Glu Leu His Ser Ala His Gly Tyr Leu Leu His Gln Phe
            180                 185                 190

ctt tct cct tct tca aac cat cgt acc gat cag tac ggc ggc agc gtg     624
Leu Ser Pro Ser Ser Asn His Arg Thr Asp Gln Tyr Gly Gly Ser Val
        195                 200                 205

gaa aat cgc gca cgt ttg gta ctg gaa gtg gtc gat gcc ggg att gaa     672
Glu Asn Arg Ala Arg Leu Val Leu Glu Val Val Asp Ala Gly Ile Glu
    210                 215                 220

gaa tgg ggt gcc gat cgc att ggc att cgc gtt tca cca atc ggt act     720
Glu Trp Gly Ala Asp Arg Ile Gly Ile Arg Val Ser Pro Ile Gly Thr
225                 230                 235                 240

ttc cag aac aca gat aac ggc ccg aat gaa gaa gcc gat gca ctg tat     768
Phe Gln Asn Thr Asp Asn Gly Pro Asn Glu Glu Ala Asp Ala Leu Tyr
                245                 250                 255

ctg att gaa caa ctg ggt aaa cgc ggc att gct tat ctg cat atg tca     816
Leu Ile Glu Gln Leu Gly Lys Arg Gly Ile Ala Tyr Leu His Met Ser
            260                 265                 270

gaa cca gat tgg gcg ggg ggt gaa ccg tat act gat gcg ttc cgc gaa     864
Glu Pro Asp Trp Ala Gly Gly Glu Pro Tyr Thr Asp Ala Phe Arg Glu
        275                 280                 285

aaa gta cgc gcc cgt ttc cac ggt ccg att atc ggc gca ggt gca tac     912
Lys Val Arg Ala Arg Phe His Gly Pro Ile Ile Gly Ala Gly Ala Tyr
    290                 295                 300

aca gta gaa aaa gct gaa acg ctg atc ggc aaa ggg tta att gat gcg     960
Thr Val Glu Lys Ala Glu Thr Leu Ile Gly Lys Gly Leu Ile Asp Ala
305                 310                 315                 320

gtg gca ttt ggt cgt gac tgg att gcg aac ccg gat ctg gtc gcc cgc    1008
Val Ala Phe Gly Arg Asp Trp Ile Ala Asn Pro Asp Leu Val Ala Arg
                325                 330                 335

ttg cag cgc aaa gct gag ctt aac cca cag cgt gcc gaa agt ttc tac    1056

Leu Gln Arg Lys Ala Glu Leu Asn Pro Gln Arg Ala Glu Ser Phe Tyr
            340                 345                 350

ggt ggc ggc gcg gaa ggc tat acc gat tac ccg acg ttg taa                1098
Gly Gly Gly Ala Glu Gly Tyr Thr Asp Tyr Pro Thr Leu
        355                 360                 365

<210>  10
<211>  365
<212>  PRT
<213>  Escherichia coli

<400>  10

Met Ser Ser Glu Lys Leu Tyr Ser Pro Leu Lys Val Gly Ala Ile Thr
1               5                   10                  15

Ala Ala Asn Arg Ile Phe Met Ala Pro Leu Thr Arg Leu Arg Ser Ile
            20                  25                  30

Glu Pro Gly Asp Ile Pro Thr Pro Leu Met Ala Glu Tyr Tyr Arg Gln
        35                  40                  45

Arg Ala Ser Ala Gly Leu Ile Ile Ser Glu Ala Thr Gln Ile Ser Ala
    50                  55                  60

Gln Ala Lys Gly Tyr Ala Gly Ala Pro Gly Ile His Ser Pro Glu Gln
65                  70                  75                  80

Ile Ala Ala Trp Lys Lys Ile Thr Ala Gly Val His Ala Glu Asn Gly
                85                  90                  95

His Met Ala Val Gln Leu Trp His Thr Gly Arg Ile Ser His Ala Ser
            100                 105                 110

Leu Gln Pro Gly Gly Gln Ala Pro Val Ala Pro Ser Ala Leu Ser Ala
        115                 120                 125

Gly Thr Arg Thr Ser Leu Arg Asp Glu Asn Gly Gln Ala Ile Arg Val
    130                 135                 140

Glu Thr Ser Met Pro Arg Ala Leu Glu Leu Glu Glu Ile Pro Gly Ile
145                 150                 155                 160

Val Asn Asp Phe Arg Gln Ala Ile Ala Asn Ala Arg Glu Ala Gly Phe
                165                 170                 175

Asp Leu Val Glu Leu His Ser Ala His Gly Tyr Leu Leu His Gln Phe
            180                 185                 190

Leu Ser Pro Ser Ser Asn His Arg Thr Asp Gln Tyr Gly Gly Ser Val
        195                 200                 205

Glu Asn Arg Ala Arg Leu Val Leu Glu Val Val Asp Ala Gly Ile Glu

210                 215                 220

Glu Trp Gly Ala Asp Arg Ile Gly Ile Arg Val Ser Pro Ile Gly Thr
225                 230                 235                 240

Phe Gln Asn Thr Asp Asn Gly Pro Asn Glu Glu Ala Asp Ala Leu Tyr
                245                 250                 255

Leu Ile Glu Gln Leu Gly Lys Arg Gly Ile Ala Tyr Leu His Met Ser
            260                 265                 270

Glu Pro Asp Trp Ala Gly Gly Glu Pro Tyr Thr Asp Ala Phe Arg Glu
        275                 280                 285

Lys Val Arg Ala Arg Phe His Gly Pro Ile Ile Gly Ala Gly Ala Tyr
    290                 295                 300

Thr Val Glu Lys Ala Glu Thr Leu Ile Gly Lys Gly Leu Ile Asp Ala
305                 310                 315                 320

Val Ala Phe Gly Arg Asp Trp Ile Ala Asn Pro Asp Leu Val Ala Arg
                325                 330                 335

Leu Gln Arg Lys Ala Glu Leu Asn Pro Gln Arg Ala Glu Ser Phe Tyr
            340                 345                 350

Gly Gly Gly Ala Glu Gly Tyr Thr Asp Tyr Pro Thr Leu
        355                 360                 365

<210>  11
<211>  1203
<212>  DNA
<213>  Saccharomyces cerevisiae

<220>
<221>  CDS
<222>  (1)..(1203)

<400>  11
atg cca ttt gtt aag gac ttt aag cca caa gct ttg ggt gac acc aac      48
Met Pro Phe Val Lys Asp Phe Lys Pro Gln Ala Leu Gly Asp Thr Asn
1               5                   10                  15

tta ttc aaa cca atc aaa att ggt aac aat gaa ctt cta cac cgt gct      96
Leu Phe Lys Pro Ile Lys Ile Gly Asn Asn Glu Leu Leu His Arg Ala
            20                  25                  30

gtc att cct cca ttg act aga atg aga gcc caa cat cca ggt aat att      144
Val Ile Pro Pro Leu Thr Arg Met Arg Ala Gln His Pro Gly Asn Ile
        35                  40                  45

cca aac aga gac tgg gcc gtt gaa tac tac gct caa cgt gct caa aga      192
Pro Asn Arg Asp Trp Ala Val Glu Tyr Tyr Ala Gln Arg Ala Gln Arg
    50                  55                  60

cca gga acc ttg att atc act gaa ggt acc ttt ccc tct cca caa tct      240
Pro Gly Thr Leu Ile Ile Thr Glu Gly Thr Phe Pro Ser Pro Gln Ser

65                  70                  75                  80

ggg ggt tac gac aat gct cca ggt atc tgg tcc gaa gaa caa att aaa      288
Gly Gly Tyr Asp Asn Ala Pro Gly Ile Trp Ser Glu Glu Gln Ile Lys
                85                  90                  95

gaa tgg acc aag att ttc aag gct att cat gag aat aaa tcg ttc gca      336
Glu Trp Thr Lys Ile Phe Lys Ala Ile His Glu Asn Lys Ser Phe Ala
            100                 105                 110

tgg gtc caa tta tgg gtt cta ggt tgg gct gct ttc cca gac acc ctt      384
Trp Val Gln Leu Trp Val Leu Gly Trp Ala Ala Phe Pro Asp Thr Leu
        115                 120                 125

gct agg gat ggt ttg cgt tac gac tcc gct tct gac aac gtg tat atg      432
Ala Arg Asp Gly Leu Arg Tyr Asp Ser Ala Ser Asp Asn Val Tyr Met
    130                 135                 140

aat gca gaa caa gaa gaa aag gct aag aag gct aac aac cca caa cac      480
Asn Ala Glu Gln Glu Glu Lys Ala Lys Lys Ala Asn Asn Pro Gln His
145                 150                 155                 160

agt ata aca aag gat gaa att aag caa tac gtc aaa gaa tac gtc caa      528
Ser Ile Thr Lys Asp Glu Ile Lys Gln Tyr Val Lys Glu Tyr Val Gln
                165                 170                 175

gct gcc aaa aac tcc att gct gct ggt gcc gat ggt gtt gaa atc cac      576
Ala Ala Lys Asn Ser Ile Ala Ala Gly Ala Asp Gly Val Glu Ile His
            180                 185                 190

agc gct aac ggt tac ttg ttg aac cag ttc ttg gac cca cac tcc aat      624
Ser Ala Asn Gly Tyr Leu Leu Asn Gln Phe Leu Asp Pro His Ser Asn
        195                 200                 205

aac aga acc gat gag tat ggt gga tcc atc gaa aac aga gcc cgt ttc      672
Asn Arg Thr Asp Glu Tyr Gly Gly Ser Ile Glu Asn Arg Ala Arg Phe
    210                 215                 220

acc ttg gaa gtg gtt gat gca gtt gtc gat gct att ggc cct gaa aaa      720
Thr Leu Glu Val Val Asp Ala Val Val Asp Ala Ile Gly Pro Glu Lys
225                 230                 235                 240

gtc ggt ttg aga ttg tct cca tat ggt gtc ttc aac agt atg tct ggt      768
Val Gly Leu Arg Leu Ser Pro Tyr Gly Val Phe Asn Ser Met Ser Gly
                245                 250                 255

ggt gct gaa acc ggt att gtt gct caa tat gct tat gtc tta ggt gaa      816
Gly Ala Glu Thr Gly Ile Val Ala Gln Tyr Ala Tyr Val Leu Gly Glu
            260                 265                 270

cta gaa aga aga gct aaa gct ggc aag cgt ttg gct ttc gtc cat cta      864
Leu Glu Arg Arg Ala Lys Ala Gly Lys Arg Leu Ala Phe Val His Leu
        275                 280                 285

gtt gaa cct cgt gtc acc aac cca ttt tta act gaa ggt gaa ggt gaa      912
Val Glu Pro Arg Val Thr Asn Pro Phe Leu Thr Glu Gly Glu Gly Glu
    290                 295                 300

tac aat gga ggt agc aac aaa ttt gct tat tct atc tgg aag ggc cca      960
Tyr Asn Gly Gly Ser Asn Lys Phe Ala Tyr Ser Ile Trp Lys Gly Pro
305                 310                 315                 320

att att aga gct ggt aac ttt gct ctg cac cca gaa gtt gtc aga gaa     1008
Ile Ile Arg Ala Gly Asn Phe Ala Leu His Pro Glu Val Val Arg Glu
                325                 330                 335

gag gtg aag gat cct aga aca ttg atc ggt tac ggt aga ttt ttt atc     1056
Glu Val Lys Asp Pro Arg Thr Leu Ile Gly Tyr Gly Arg Phe Phe Ile

340                 345                 350

tct aat cca gat ttg gtt gat cgt ttg gaa aaa ggg tta cca tta aac        1104
Ser Asn Pro Asp Leu Val Asp Arg Leu Glu Lys Gly Leu Pro Leu Asn
        355                 360                 365

aaa tat gac aga gac act ttc tac aaa atg tca gct gag gga tac att        1152
Lys Tyr Asp Arg Asp Thr Phe Tyr Lys Met Ser Ala Glu Gly Tyr Ile
    370                 375                 380

gac tac cct acg tac gaa gaa gct cta aaa ctc ggt tgg gac aaa aat        1200
Asp Tyr Pro Thr Tyr Glu Glu Ala Leu Lys Leu Gly Trp Asp Lys Asn
385                 390                 395                 400

taa                                                                    1203

<210>  12
<211>  400
<212>  PRT
<213>  Saccharomyces cerevisiae

<400>  12

Met Pro Phe Val Lys Asp Phe Lys Pro Gln Ala Leu Gly Asp Thr Asn
1               5                   10                  15

Leu Phe Lys Pro Ile Lys Ile Gly Asn Asn Glu Leu Leu His Arg Ala
            20                  25                  30

Val Ile Pro Pro Leu Thr Arg Met Arg Ala Gln His Pro Gly Asn Ile
        35                  40                  45

Pro Asn Arg Asp Trp Ala Val Glu Tyr Tyr Ala Gln Arg Ala Gln Arg
    50                  55                  60

Pro Gly Thr Leu Ile Ile Thr Glu Gly Thr Phe Pro Ser Pro Gln Ser
65                  70                  75                  80

Gly Gly Tyr Asp Asn Ala Pro Gly Ile Trp Ser Glu Glu Gln Ile Lys
                85                  90                  95

Glu Trp Thr Lys Ile Phe Lys Ala Ile His Glu Asn Lys Ser Phe Ala
            100                 105                 110

Trp Val Gln Leu Trp Val Leu Gly Trp Ala Ala Phe Pro Asp Thr Leu
        115                 120                 125

Ala Arg Asp Gly Leu Arg Tyr Asp Ser Ala Ser Asp Asn Val Tyr Met
    130                 135                 140

Asn Ala Glu Gln Glu Glu Lys Ala Lys Lys Ala Asn Asn Pro Gln His
145                 150                 155                 160

Ser Ile Thr Lys Asp Glu Ile Lys Gln Tyr Val Lys Glu Tyr Val Gln
                165                 170                 175

Ala Ala Lys Asn Ser Ile Ala Ala Gly Ala Asp Gly Val Glu Ile His
            180                 185                 190

Ser Ala Asn Gly Tyr Leu Leu Asn Gln Phe Leu Asp Pro His Ser Asn
        195                 200                 205

Asn Arg Thr Asp Glu Tyr Gly Gly Ser Ile Glu Asn Arg Ala Arg Phe
    210                 215                 220

Thr Leu Glu Val Val Asp Ala Val Val Asp Ala Ile Gly Pro Glu Lys
225                 230                 235                 240

Val Gly Leu Arg Leu Ser Pro Tyr Gly Val Phe Asn Ser Met Ser Gly
                245                 250                 255

Gly Ala Glu Thr Gly Ile Val Ala Gln Tyr Ala Tyr Val Leu Gly Glu
            260                 265                 270

Leu Glu Arg Arg Ala Lys Ala Gly Lys Arg Leu Ala Phe Val His Leu
        275                 280                 285

Val Glu Pro Arg Val Thr Asn Pro Phe Leu Thr Glu Gly Glu Gly Glu
    290                 295                 300

Tyr Asn Gly Gly Ser Asn Lys Phe Ala Tyr Ser Ile Trp Lys Gly Pro
305                 310                 315                 320

Ile Ile Arg Ala Gly Asn Phe Ala Leu His Pro Glu Val Val Arg Glu
                325                 330                 335

Glu Val Lys Asp Pro Arg Thr Leu Ile Gly Tyr Gly Arg Phe Phe Ile
            340                 345                 350

Ser Asn Pro Asp Leu Val Asp Arg Leu Glu Lys Gly Leu Pro Leu Asn
        355                 360                 365

Lys Tyr Asp Arg Asp Thr Phe Tyr Lys Met Ser Ala Glu Gly Tyr Ile
    370                 375                 380

Asp Tyr Pro Thr Tyr Glu Glu Ala Leu Lys Leu Gly Trp Asp Lys Asn
385                 390                 395                 400

<210>  13
<211>  1017
<212>  DNA
<213>  Bacillus subtilis

<220>
<221>  CDS
<222>  (1)..(1017)

<400>  13
atg gcc aga aaa tta ttt aca cct att aca att aaa gat atg acg tta      48

Met Ala Arg Lys Leu Phe Thr Pro Ile Thr Ile Lys Asp Met Thr Leu
1               5                   10                  15

aaa aac cgc att gtc atg tcg cca atg tgc atg tat tct tct cat gaa      96
Lys Asn Arg Ile Val Met Ser Pro Met Cys Met Tyr Ser Ser His Glu
            20                  25                  30

aag gac gga aaa tta aca ccg ttc cac atg gca cat tac ata tcg cgc      144
Lys Asp Gly Lys Leu Thr Pro Phe His Met Ala His Tyr Ile Ser Arg
        35                  40                  45

gca atc ggc cag gtc gga ctg att att gta gag gcg tca gcg gtt aac      192
Ala Ile Gly Gln Val Gly Leu Ile Ile Val Glu Ala Ser Ala Val Asn
    50                  55                  60

cct caa gga cga atc act gac caa gac tta ggc att tgg agc gac gag      240
Pro Gln Gly Arg Ile Thr Asp Gln Asp Leu Gly Ile Trp Ser Asp Glu
65                  70                  75                  80

cat att gaa ggc ttt gca aaa ctg act gag cag gtc aaa gaa caa ggt      288
His Ile Glu Gly Phe Ala Lys Leu Thr Glu Gln Val Lys Glu Gln Gly
                85                  90                  95

tca aaa atc ggc att cag ctt gcc cat gcc gga cgt aaa gct gag ctt      336
Ser Lys Ile Gly Ile Gln Leu Ala His Ala Gly Arg Lys Ala Glu Leu
            100                 105                 110

gaa gga gat atc ttc gct cca tcg gcg att gcg ttt gac gaa caa tca      384
Glu Gly Asp Ile Phe Ala Pro Ser Ala Ile Ala Phe Asp Glu Gln Ser
        115                 120                 125

gca aca cct gta gaa atg tca gca gaa aaa gta aaa gaa acg gtc cag      432
Ala Thr Pro Val Glu Met Ser Ala Glu Lys Val Lys Glu Thr Val Gln
    130                 135                 140

gag ttc aag caa gcg gct gcc cgc gca aaa gaa gcc ggc ttt gat gtg      480
Glu Phe Lys Gln Ala Ala Ala Arg Ala Lys Glu Ala Gly Phe Asp Val
145                 150                 155                 160

att gaa att cat gcg gcg cac gga tat tta att cat gaa ttt ttg tct      528
Ile Glu Ile His Ala Ala His Gly Tyr Leu Ile His Glu Phe Leu Ser
                165                 170                 175

ccg ctt tcc aac cat cga aca gat gaa tat ggc ggc tca cct gaa aac      576
Pro Leu Ser Asn His Arg Thr Asp Glu Tyr Gly Gly Ser Pro Glu Asn
            180                 185                 190

cgc tat cgt ttc ttg aga gag atc att gat gaa gtc aaa caa gta tgg      624
Arg Tyr Arg Phe Leu Arg Glu Ile Ile Asp Glu Val Lys Gln Val Trp
        195                 200                 205

gac ggt cct tta ttt gtc cgt gta tct gct tct gac tac act gat aaa      672
Asp Gly Pro Leu Phe Val Arg Val Ser Ala Ser Asp Tyr Thr Asp Lys
    210                 215                 220

ggc tta gac att gcc gat cac atc ggt ttt gca aaa tgg atg aag gag      720
Gly Leu Asp Ile Ala Asp His Ile Gly Phe Ala Lys Trp Met Lys Glu
225                 230                 235                 240

cag ggt gtt gac tta att gac tgc agc tca ggc gcc ctt gtt cac gca      768
Gln Gly Val Asp Leu Ile Asp Cys Ser Ser Gly Ala Leu Val His Ala
                245                 250                 255

gac att aac gta ttc cct ggc tat cag gtc agc ttc gct gag aaa atc      816
Asp Ile Asn Val Phe Pro Gly Tyr Gln Val Ser Phe Ala Glu Lys Ile
            260                 265                 270

cgt gaa cag gcg gac atg gct act ggt gcc gtc ggc atg att aca gac      864

Arg Glu Gln Ala Asp Met Ala Thr Gly Ala Val Gly Met Ile Thr Asp
        275                 280                 285

ggt tca atg gct gaa gaa att ctg caa aac gga cgt gcc gac ctc atc 912
Gly Ser Met Ala Glu Glu Ile Leu Gln Asn Gly Arg Ala Asp Leu Ile
    290                 295                 300

ttt atc ggc aga gag ctt ttg cgg gat cca ttt ttt gca aga act gct 960
Phe Ile Gly Arg Glu Leu Leu Arg Asp Pro Phe Phe Ala Arg Thr Ala
305                 310                 315                 320

gcg aaa cag ctc aat aca gag att ccg gcc cct gtt caa tac gaa aga 1008
Ala Lys Gln Leu Asn Thr Glu Ile Pro Ala Pro Val Gln Tyr Glu Arg
                325                 330                 335

ggc tgg taa 1017
Gly Trp

<210> 14
<211> 338
<212> PRT
<213> Bacillus subtilis

<400> 14

Met Ala Arg Lys Leu Phe Thr Pro Ile Thr Ile Lys Asp Met Thr Leu
1               5                   10                  15

Lys Asn Arg Ile Val Met Ser Pro Met Cys Met Tyr Ser Ser His Glu
            20                  25                  30

Lys Asp Gly Lys Leu Thr Pro Phe His Met Ala His Tyr Ile Ser Arg
        35                  40                  45

Ala Ile Gly Gln Val Gly Leu Ile Ile Val Glu Ala Ser Ala Val Asn
    50                  55                  60

Pro Gln Gly Arg Ile Thr Asp Gln Asp Leu Gly Ile Trp Ser Asp Glu
65                  70                  75                  80

His Ile Glu Gly Phe Ala Lys Leu Thr Glu Gln Val Lys Glu Gln Gly
                85                  90                  95

Ser Lys Ile Gly Ile Gln Leu Ala His Ala Gly Arg Lys Ala Glu Leu
            100                 105                 110

Glu Gly Asp Ile Phe Ala Pro Ser Ala Ile Ala Phe Asp Glu Gln Ser
        115                 120                 125

Ala Thr Pro Val Glu Met Ser Ala Glu Lys Val Lys Glu Thr Val Gln
    130                 135                 140

Glu Phe Lys Gln Ala Ala Ala Arg Ala Lys Glu Ala Gly Phe Asp Val
145                 150                 155                 160

Ile Glu Ile His Ala Ala His Gly Tyr Leu Ile His Glu Phe Leu Ser

                165                 170                 175

Pro Leu Ser Asn His Arg Thr Asp Glu Tyr Gly Gly Ser Pro Glu Asn
            180                 185                 190

Arg Tyr Arg Phe Leu Arg Glu Ile Ile Asp Glu Val Lys Gln Val Trp
        195                 200                 205

Asp Gly Pro Leu Phe Val Arg Val Ser Ala Ser Asp Tyr Thr Asp Lys
    210                 215                 220

Gly Leu Asp Ile Ala Asp His Ile Gly Phe Ala Lys Trp Met Lys Glu
225                 230                 235                 240

Gln Gly Val Asp Leu Ile Asp Cys Ser Ser Gly Ala Leu Val His Ala
                245                 250                 255

Asp Ile Asn Val Phe Pro Gly Tyr Gln Val Ser Phe Ala Glu Lys Ile
            260                 265                 270

Arg Glu Gln Ala Asp Met Ala Thr Gly Ala Val Gly Met Ile Thr Asp
        275                 280                 285

Gly Ser Met Ala Glu Glu Ile Leu Gln Asn Gly Arg Ala Asp Leu Ile
    290                 295                 300

Phe Ile Gly Arg Glu Leu Leu Arg Asp Pro Phe Phe Ala Arg Thr Ala
305                 310                 315                 320

Ala Lys Gln Leu Asn Thr Glu Ile Pro Ala Pro Val Gln Tyr Glu Arg
                325                 330                 335

Gly Trp

<210> 15
<211> 1212
<212> DNA
<213> Artificial

<220>
<223> codon optimised OYE gene from Candida macedoniensis

<400> 15
atgtcctaca tgaactttga cccgaaaccg ctgggggata ccaacatctt caaaccgatt 60

aaaatcggta ataacgaact gaagcaccgt gttgtaatgc cggcgctgac gcgtatgcgt 120

gcaattgccc cgggtaacat tccgaacacc gaatgggctg aagaatacta tcgccagcgt 180

tctcagtacc cgggcactct gatcatcacc gaaggcacct tcccgtccgc gcagtctggt 240

ggttatccga atgttccggg tatttggtct aaggaacagc tggctgaatg gaagaaaatc 300

ttcaatgcga ttcacgaaaa caaaagcttc gtttgggttc aactgtgggt gctgggccgt 360

caggcttggc cggaagttct gaagaaagaa gggctgcgtt atgactccgc gaccgatgac 420

ctgtatatgg gtgaggaaga aaaagaacgc gcactgaaag caaacaaccc acaacacggg 480

attaccaagg aagaaattaa acagtacatc aaagagtatg ttgatgcggc gaaaaaggct 540

atcgacgccg gtgctgacgg cgtgcagatc cattccgcca acggttacct gctgaaccag 600

ttcctggacc cgatctccaa taaccgtacc gatgaatacg gtggctctat cgaaaatcgc 660

gctcgtttca ccctggaagt agttgacgcc gttgttgacg ccgtgggcgc ggagcgtacc 720

tccatccgct tctctccgta cggaacgttc ggtactatgt ccggaggcga gaacccggga 780

atcgtggctc aatacgctta cgttattggt gaactggaga aacgcgcacg cgctggtaaa 840

cgcctggcgt tcatcgatct ggtggaaccg cgcgttactg atccgttcct gccggagttt 900

gaaaaatggt ttaaagaagg taccaacgag ttcatctaca gcatttggaa aggtccggta 960

ctgcgtgttg gcaattatgc cctggacccg gaccaggcga ctctggatag caaaaagccg 1020

aacacgctga ttggctatgg tcgcagcttc atcgccaatc cagacctggt gtaccgcctg 1080

gaaaaaggac tgcctctgaa caaatatgac cgcaacactt tctacacttt cactaaggaa 1140

ggatacactg actacccgtc ctacgaggaa tctgtagcaa aagggtataa aaaggaagaa 1200

aagaaatact aa 1212

<210> 16
<211> 1197
<212> DNA
<213> Artificial

<220>
<223> codon optimised KYE1 gene from Kluyveromyces lactis NRRL Y-1140

<400> 16
atgagcttta tgaatttcga gcctaaaccg ctggccgata ctgatatctt caagccgatc 60

aaaattggta ataccgagct gaaacaccgt gtagttatgc cggctctgac tcgtatgcgc 120

gcgctgcatc caggtaacgt gccgaacccg gactgggctg ttgaatacta ccgtcagcgt 180

tcccaatatc ctggcaccat gatcatcact gaaggtgctt ttccgtctgc ccagtctggt 240

ggctacgata atgctcctgg cgtttggtct gaggaacaac tggcccagtg gcgcaagatc 300

tttaaagcga tccatgacaa caaaagcttc gtctgggtac agctgtgggt tctgggtcgt 360

caggcgttcg cagataacct ggctcgcgac ggtctgcgtt acgattctgc ctccgacgaa 420

gtttacatgg gtgaagatga gaaagaacgc gccatccgtt ccaacaaccc gcaacacggt 480

atcacgaaag atgaaatcaa gcagtacatc cgtgactacg tggacgcagc taaaaagtgt 540

atcgacgcgg gcgctgatgg tgttgaaatt cactctgcaa acggctacct gctgaaccag 600

tttctggacc ctatctccaa caaacgtact gacgaatatg gcggttctat cgagaatcgt 660

gcccgcttcg tactggaagt agttgacgcg gttgtggacg cggtgggcgc ggagcgtacc 720

agcattcgtt tcagcccata tggcgtattc ggtactatgt ccggtgtttc cgacccggtt 780

ctggttgcac agttcgcata tgtcctggcg gaactggaaa aacgtgcaaa agctggtaaa 840

cgtctggcat acgttgacct ggtggaaccg cgtgtaacct ccccattcca gccggaattt 900
gaaggctggt ataaaggcgg caccaacgaa ttcgtataca gcgtttggaa aggtaacgtt 960
ctgcgcgttg gcaactacgc tctggatccg gacgctgcaa ttaccgactc taaaaacccg 1020
aataccctga tcggctacgg ccgtgctttc atcgctaacc ctgatctggt agaacgtctg 1080
gagaaaggcc tgcctctgaa ccagtacgat cgtccttcct tttacaaaat gtccgccgag 1140
ggttatatcg attatccgac ttatgaagaa gcggttgcta agggctataa aaagtaa 1197

<210> 17
<211> 1050
<212> DNA
<213> Artificial

<220>
<223> codon optimised xenB gene from Pseudomonas fluorescens I-C

<400> 17
atggcgacta tttttgaccc gattaaactg ggagatctgg aactgtctaa ccgcattatc 60
atggcgccgc tgacccgttg ccgtgcggat gagggccgcg ttccaaatgc tctgatggct 120
gagtactatg tgcagcgtgc gtccgcaggt ctgattctgt ctgaagcgac ctccgtgact 180
cctatggggg taggctaccc ggatacgccg ggtatttggt ccaatgacca ggtgcgtgga 240
tggacgaaca tcaccaaagc tgttcacgca gcaggcggta agatcgttct gcaactgtgg 300
cacgttggcc gtatttccca cccgctgtac ctgaacggag aggcgccggt agctccgtct 360
gctatccaac cgaaaggtca tgtatccctg gtgcgtccgc tggctgatta tcctactccg 420
cgtgcgctgg aaaccgcgga gattgcggaa atcgttgaag catatcgtac gggagccgaa 480
aacgcgaaag ctgcgggctt tgacggcgtc gaaatccatg gcgctaacgg ttatctgctg 540
gatcagtttc tgcaaagctc cacgaatcag cgtaccgaca actacggcgg atctctggaa 600
aaccgtgcgc gcctgctgct ggaagtaacc gacgccgcaa ttgatgtgtg gggagccggc 660
cgtgttggtg ttcacctggc tcctcgtgcg gatagccacg acatgggtga cgacaacctg 720
gcagaaacct tcacttatgt ggctcgcgaa ctgggaaaac gcgggatcgc attcatctgt 780
tctcgcgaaa aagaaggtgc cgactccctg ggcccgcaac tgaaggaagc ctttggaggc 840
gcttatattg cgaacgagcg ttttaccaaa gattctgcaa acgcgtggct ggcggaaggt 900
aaagcagatg cagttgcgtt tggcgttccg ttcattgcca accctgacct gccggctcgc 960
ctgaaggcag acgctcctct gaacgaacct cgcccggaac tgttctacgg caaaggtccg 1020
gtgggatata tcgattaccc gaccctgtaa 1050

<210> 18
<211> 1083
<212> DNA
<213> Artificial

<220>
<223> codon optimised ncr gene from Pseudomonas syringae pv. glycinea

<400> 18

atgccaactc tgtttgaccc gctgactctg ggcgacctgc aatccccgaa ccgtgttctg 60
atggctcctc tgacccgtgg acgtgccacc cgtgagcacg tgccgaccga gctgatgatc 120
gaatactata cccagcgcgc gagcgcaggc ctgatcatca ccgaagccac gggcatcacc 180
caggaaggtc tgggctggcc gtatgctccg gggatctggt ccgacgagca ggtggaagcc 240
tggaaaccgg tgactcaggc tgtacatgaa gcgggtggac gcattatcct gcaactgtgg 300
cacatgggtc gcaccgttca ttcttctttc ctgggtggcg caaaacctgt ttctagctcc 360
gcaacccgtg caccgggaca ggctcacact tacgaaggga aacaggatta cgacgaagcg 420
cgtccgctga gcgccgatga aattccgcgt ctgctgaacg attatgagca cgctgccaaa 480
aacgcaatgg cagcaggctt cgatggtgtc cagattcacg ccgctaacgg ctatctgatc 540
gaccaatttc tgcgtgataa ctccaacgtt cgcggggacg catacggtgg ctctatcgag 600
aaccgtattc gcctgctggt tgaagttacc cgtcgtgttg ccgaaaccgt gggagccgag 660
aaaaccggtg tgcgtctgtc cccgaatggc gactcccagg gggtgaacga ttctaaccca 720
gaaccgctgt tttctgcggc ggctaaagca ctggatgaga ttggtatcgc ccacctggaa 780
ctgcgtgaac cgggctatga aggcactttt ggcaaagctg accgtccgcc ggtacatcca 840
gtcatccgtc aggcattttc tcgtaccctg atcctgaaca gcgattacac gctggaaacc 900
gcacaggctg cactggcaac cggtgaagct gatgctatta cttttggccg tccgtttctg 960
gcgaaccctg acctgccgca tcgtttcgcg gaacgtctgc cgctgaacaa ggatgttatg 1020
gaaacctggt actctcaggg accggagggt tacgtagatt atccaaccgc agatcagaaa 1080
taa 1083

## Description

The invention relates to a method for preparing (Z)-6,7-dihydro-1*H-*azepin-2(5*H*)-one (herein after also referred to as 6,7-DAO). The invention further relates to a method for preparing ε-caprolactam (hereafter referred to as 'caprolactam') from 6,7-DAO. The invention further relates to a host cell which may be used in the preparation of 6,7-DAO.

Caprolactam is a lactam which may be used for the production of polyamide, for instance nylon-6 or nylon-6,6. Various manners of preparing caprolactam from bulk chemicals are known in the art and include the preparation of caprolactam from cyclohexanone, toluene, phenol, cyclohexanol, benzene or cyclohexane. These intermediate compounds are generally obtained from mineral oil. In view of a growing desire to prepare materials using more sustainable technology it would be desirable to provide a method wherein caprolactam is prepared from an intermediate compound that can be obtained from a biological source or at least from an intermediate compound that is converted into caprolactam using a biochemical method. Furthermore, it would be desirable to provide a method that requires less energy than conventional chemical processes making used of bulk chemicals from petrochemical origin.

It is known to prepare caprolactam from 6-aminocaproic acid (6-ACA), *e.g*. as described in US-A 6,194,572. As disclosed in WO 2005/068643, 6-ACA may be prepared biochemically by converting 6-aminohex-2-enoic acid (6-AHEA) in the presence of an enzyme having α,β-enoate reductase activity. The 6-AHEA may be prepared from lysine, *e.g*. biochemically or by pure chemical synthesis. Although, the preparation of 6-ACA via the reduction of 6-AHEA is feasible by the methods disclosed in WO 2005/068643, the inventors have found that - under the reduction reaction conditions - 6-AHEA may spontaneously and substantially irreversibly cyclise to form an undesired side-product, notably β-homoproline. This cyclisation may be a bottle neck in the production of 6-ACA, and lead to a considerable loss in yield.

It is an object of the invention to provide a novel method for preparing caprolactam that can serve as an alternative for known methods. It is in particular an object to provide a novel method for preparing an intermediate compound that can be used to prepare caprolactam.

It is a further object to provide a novel method that would overcome one or more of the drawbacks mentioned above.

It is a further object to provide a novel fermentative method for preparing caprolactam or an intermediate compound for preparing caprolactam.

One or more further objects which may be solved in accordance with the invention, will follow from the description, below.

It has now been found possible to prepare caprolactam or an intermediate compound for preparing caprolactam, biochemically from a specific starting compound.

Accordingly, the invention relates to a method for preparing (Z)-6,7-dihydro-1*H*-azepin-2(5*H*)-one (6,7-DAO) comprising removing the α-amino group of α-amino-ε-caprolactam (ACL), wherein the removal is catalysed by a biocatalyst.

The invention further relates to a method for preparing ε-caprolactam comprising reducing the carbon-carbon double bond of 6,7-DAO prepared in a method according to the invention.

The invention is based on the insight that it is possible to prepare caprolactam biocatalytically from 6,7-DAO and that 6,7-DAO can be prepared from α-amino-ε-caprolactam (ACL), by removing the α-aminogroup, *e.g*. by ammonia elimination, or by subsequent transamination, keto-group reduction and dehydration, catalysed by a biocatalyst.

The invention allows the preparation of an intermediate compound - *i*.*e*. 6,7-DAO - suitable for preparing caprolactam, that is essentially free of undesired cyclic side-product, in particular β-homoproline.

In an advantageous embodiment of the invention 6,7-DAO or caprolactam is prepared fermentatively.

The term "or" as used herein means "and/or" unless specified otherwise.

The term "a" or "an" as used herein means "at least one" unless specified other wise.

When referring to a moiety (*e*.*g*. a compound, an additive *etc.*) in singular, the plural is meant to be included. Thus, when referring to a specific moiety, *e*.*g*. "compound", this means "at least one" of that moiety, *e.g*. "at least one compound", unless specified otherwise.

When referred to a compound of which stereisomers exist, the compound may be any of such stereoisomers or a combination thereof. Thus, when referred to, *e.g.*, ACL or an amino acid of which enantiomers exist, the ACL or the amino acid may be the L-enantiomer, the D-enantiomer or a combination thereof. In case a natural stereoisomer exists, the compound is preferably a natural stereoisomer.

When referred herein to a carboxylic acid or a carboxylate, *e*.*g*. an amino acid these terms are meant to include the protonated carboxylic acid group, the corresponding carboxylate (its conjugated base) as well as a salt thereof. Likewise, when referring to an amine (*e*.*g*. lysine or another amino acid, or ACL), this is meant to include the protonated amine (typically cationic, *e*.*g*. R-NH₃⁺) and the unprotonated amine (typically uncharged, *e*.*g*. R-NH₂).

When an enzyme is mentioned with reference to an enzyme class (EC) between brackets, the enzyme class is a class wherein the enzyme is classified or may be classified, on the basis of the Enzyme Nomenclature provided by the Nomenclature Committee of the International Union of Biochemistry and Molecular Biology (NC-IUBMB), which nomenclature may be found at http://www.chem.qmul.ac.uk/iubmb/enzyme/. Other suitable enzymes that have not (yet) been classified in a specified class but may be classified as such, are meant to be included.

The term "homologue" is used herein in particular for polynucleotides or polypeptides having a sequence identity of at least 30 %, preferably at least 40 %, more preferably at least 60%, more preferably at least 65%, more preferably at least 70 %, more preferably at least 75%, more preferably at least 80%, in particular at least 85 %, more in particular at least 90 %, at least 91 %, at least 92 %, at least 93 %, at least 94 %, at least 95 %, at least 96 %, at least 97 %, at least 98 % or at least 99 %. The term homologue is also meant to include polynucleotide sequences which differ from another polynucleotide sequence due to the degeneracy of the genetic code and encode the same polypeptide sequence.

Sequence identity or similarity is herein defined as a relationship between two or more polypeptide sequences or two or more polynucleotide sequences, as determined by comparing the sequences. Usually, sequence identities or similarities are compared over the whole length of the sequences, but may however also be compared only for a part of the sequences aligning with each other. In the art, "identity" or "similarity" also means the degree of sequence relatedness between polypeptide sequences or polynucleotide sequences, as the case may be, as determined by the match between strings of such sequences. Preferred methods to determine identity or similarity are designed to give the largest match between the sequences tested. In context of this invention a preferred computer program method to determine identity and similarity between two sequences includes BLASTP and BLASTN (Altschul, S. F. et al., J. Mol. Biol. 1990, 215, 403-410, publicly available from NCBI and other sources (BLAST Manual, Altschul, S., et al., NCBI NLM NIH Bethesda, MD 20894). Preferred parameters for polypeptide sequence comparison using BLASTP are gap open 10.0, gap extend 0.5, Blosum 62 matrix. Preferred parameters for nucleotide sequence comparison using BLASTN are gap open 10.0, gap extend 0.5, DNA full matrix (DNA identity matrix).

In a method of the invention, a biocatalyst is used, *i.e.* at least one reaction step in the method is catalysed by a biological material or moiety derived from a biological source, for instance an organism or a biomolecule derived there from. The biocatalyst may in particular comprise one or more enzymes. The biocatalyst may be used in any form. In an embodiment, one or more enzymes are used isolated from the natural environment (isolated from the organism it has been produced in), for instance as a solution, an emulsion, a dispersion, as a lysate, or immobilised on a support. In an embodiment, one or more enzymes form part of a living organism (such as living whole cells). The enzymes may perform a catalytic function inside the cell. It is also possible that the enzyme may be secreted into a medium, wherein the cells are present.

Living cells may be growing cells, resting or dormant cells (*e.g*. spores) or cells in a stationary phase. It is also possible to use an enzyme forming part of a permeabilised cell (*i.e.* made permeable to a substrate for the enzyme or a precursor for a substrate for the enzyme or enzymes).

A biocatalyst used in a method of the invention may in general be any organism, or be obtained or derived from any organism. The organism may be eukaryotic or prokaryotic. In particular the organism may be selected from animals (including humans), plants, bacteria, archaea, yeasts and fungi.

In an embodiment a biocatalyst, *e.g*. and enzyme, may originate from an animal, in particular from a part thereof - *e.g*. liver, pancreas, brain, kidney or other organ. The animal may in particular be selected from invertebrate marine animals, more in particular sponges (*Porifera*), in particular from *Demospongiae, Pachastrellidae* or *Jaspidae, e.g. Jaspis sp., Pachastrella* sp., *Poecillastra sollasi, Choristidae* and mammals, more in particular mammals selected from the group of *Leporidae, Muridae*, *Suidae, and Bovidae*. Suitable bacteria may in particular be selected amongst the group of *Pseudomonas, Bacillus, Escherichia, Ochrobactrum, Citrobacter; Klebsiella, Mycobacterium, Providencia, Achromobacter, Rhodococcus, Myxococcus, Enterobacter, Methylophilus, Streptomyces, Nocardia, Thermus* and *Alcaligenes*.

Suitable fungi may in particular be selected amongst the group of *Aspergillus, Tremella* and *Periconia*.

Suitable yeasts may in particular be selected amongst the group of *Candida, Saccharomyces, Kluyveromyces, Cryptococcus* and *Trichosporon*. It will be clear to the person skilled in the art that use can be made of a naturally occurring biocatalyst (wild type) or a mutant of a naturally occurring biocatalyst with suitable activity in a method according to the invention. Properties of a naturally occurring biocatalyst may be improved by biological techniques known to the skilled person in the art, such as *e.g*. molecular evolution or rational design. Mutants of wild-type biocatalysts can for example be made by modifying the encoding DNA of an organism capable of acting as a biocatalyst or capable of producing a biocatalytic moiety (such as an enzyme) using mutagenesis techniques known to the person skilled in the art (random mutagenesis, site-directed mutagenesis, directed evolution, gene recombination, *etc*.). In particular the DNA may be modified such that it encodes an enzyme that differs by at least one amino acid from the wild-type enzyme, so that it encodes an enzyme that comprises one or more amino acid substitutions, deletions and/or insertions compared to the wild-type, or such that the mutants combine sequences of two or more parent enzymes or by effecting the expression of the thus modified DNA in a suitable (host) cell. The latter may be achieved by methods known to the skilled person in the art such as codon optimisation or codon pair optimisation, *e.g*. based on a method as described in WO 2008/000632. WO 2003/010183 discloses a particularly suitable process for the preparation of variant polynucleotides using a combination of mutagenesis of a starting population of polynucleotides and recombination of the mutated polynucleotides.

A mutant biocatalyst may have improved properties, for instance with respect to one or more of the following aspects: selectivity towards the substrate, activity, stability, solvent tolerance, pH profile, temperature profile, substrate profile, susceptibility to inhibition, cofactor utilisation and substrate-affinity. Mutants with improved properties can be identified by applying *e.g*. suitable high throughput screening or selection methods based on such methods known to the skilled person in the art.

When referred to a biocatalyst, in particular an enzyme, from a particular source, recombinant biocatalysts, in particular enzymes, originating from a first organism, but actually produced in a (genetically modified) second organism, are specifically meant to be included as biocatalysts, in particular enzymes, from that first organism.

ACL used in a method according to the invention can in principle be obtained in any way. For instance, ACL may be synthesised chemically, or biocatalytically. In an embodiment ACL is extracted from a natural source. For instance ACL may be obtained by, *e.g*. mild acidic, hydrolysis of an ACL moiety from a naturally occurring molecule comprising such moiety. For instance, naturally occurring molecules from which ACL may be obtained after hydrolysis are capuramycin from *Streptomyces griseus*; bengamide and isobengamide derivatives from sponges belonging to the *Jaspidae*; sesquiterpene derivatives from the sponge *Poechillastra solassi*; bengamide derivatives from *Myxococcus virescens*; caprolactin A and B from the deep sea isolate PC12/1000-B4 (ref.: Tetrahedron **1993,** *49*(30), 6569-6574); nocardiamycin derivatives from *Nocardia* sp.; and circinatin from the fungus *Periconia circinata*.

In an embodiment of the invention, ACL is prepared by cyclising lysine, in the presence of a biocatalyst. In principle, D-lysine, L-lysine or a mixture thereof can be used. By cyclising these, D-ACL, L-ACL or a mixture thereof is formed. In practice, L-lysine is preferred.

A biocatalyst used in this cyclisation reaction, preferably comprises an enzyme having lysine cyclase activity. For instance an enzyme having lysine cyclase activity may be used originating from an organism as identified above.

In particular, an enzyme capable of catalysing the cyclisation of lysine to ACL, may be selected from the group of hydrolases (EC 3). The hydrolase preferably is selected from the group of hydrolases acting on ester bonds (esterases) (EC 3.1), and hydrolytic enzymes acting upon carbon-nitrogen bonds, other than peptide bonds (EC 3.5). An esterase may in particular be selected from the group of carboxylic ester hydrolases (EC 3.1.1) and more in particular carboxyl esterases (EC 3.1.1.1), preferably from pig liver esterases. An enzyme of EC class 3.5 may in particular be selected from the group of hydrolases mainly acting on linear amides (EC 3.5.1).

A hydrolase mainly acting on linear amides, such as an amidase, may in particular be such hydrolase from *Ochrobactrum, Rhodococcus, Enterobacter*, *Thermus, Klebsiella, Aspergillus, Methylophilus* or *Mycobacterium*. More in particular a hydrolase mainly acting on linear amides, such as an amidase, may be used from an organism selected from the group of *Ochrobactrum anthropi, Rhodococcus erythropolis, Enterobacter cloacae, Thermus sp., Klebsiella terrigena, Klebsiella oxytoca, Aspergillus nidulans, Methylophilus methylotrophus* and *Mycobacterium smegmatis*.

An amidase originating from *Ochrobactrum anthropi* NCIMB 40321 or an amidase originating from *Rhodococcus erythropolis* NCIMB 11540 is particularly advantageous in a method wherein ACL is further used for the preparation of caprolactam. Further, an amidase may be used as described in US 2005/0079595 or in EP-A 1 409 667 for the cyclisation reaction, the contents of which with respect to enzymes having lysine cyclase activity and genes coding for such enzymes are incorporated herein by reference.

Furthermore, an enzyme of EC class 3.5 may also in particular be selected from the group of hydrolases mainly acting on C-N bonds in cyclic amides (EC 3.5.2), which may also be referred to as a lactamase, and more in particular be selected from the group of lysine lactamases (EC 3.5.2.11).

In particular, a lactamase (*i.e.* a hydrolase acting in cyclic amides) may be selected amongst L-lysine-1,6-lactam hydrolases (EC 3.5.2.11) and 6-aminohexanoate-cyclic dimer hydrolases (EC 3.5.2.12).

In an embodiment a lactamase, in particular an L-lysine lactamases, is selected amongst the group of lactamases from *Aspergillus, Cryptococcus, Candida, Citrobacter, Trichosporon, Tremella* and *Providencia*. More in particular, said lactamase may be selected amongst the group of lactamases originating from *Aspergillus ustus, Aspergillus niger, Cryptococcus laurentii, Candida humicola, Citrobacter freundii, Trichosporon cutaneum, Tremella fuciformis, Tremella aurentia, Tremella foliacea, Tremella subanomalia and Providencia alcalifaciens*.

In an embodiment the lactamase, in particular a 6-aminohexanoatecyclic dimer hydrolase (EC 3.5.2.12) is a lactamase from *Alcaligenes*, such as from *Alcaligenes lactamlytics* or from *Achromobacter*, such as from *Achromobacterxerosis* or from *Achromobacter guttatus*.

A lipase may in particular be selected from lipases originating from a mammal, such as porcine lipase, bovine lipase or the like. In particular, a lipase used in a method of the invention may be a pancreatic lipase. Lipases are commercially available, *e.g*. porcine pancreas lipase may be obtained from Röhm (catalogue number 7023C) or from Sigma (catalogue number L-3126). It is known to the person skilled in the art that commercial pig liver esterase (PLE) preparations, *e.g*. available from Sigma as a suspension (catalog number E2884) or in powder form (catalog number E3019), usually are a mixture of enzymes, amongst others, isoenzymes, of pig liver esterase. It is contemplated that one or more of these isoenzymes in the PLE preparation are responsible for the bioconversion of lysine to ACL. A person skilled in the art knows how to isolate, clone and/or express a pig liver esterase isoenzyme into a suitable host, if desired.

In an embodiment, one may use a non-ribosomal peptide synthase (NRPS) for cyclisation of lysine. It is known for secondary metabolite producers to synthesise peptides via non-ribosomal peptide synthases (NRPSs). NRPSs are in detail described in, *e.g*., "Assembly-Line Enzymology for Polyketide and Nonribosomal Peptide Antibiotics: Logic, Machinery, and Mechanisms", Michael A. Fischbach and Christopher T. Walsh, Chem. Rev. 2006, 106, 3468-3496, and in WO/00/58478. In some instances biocatalysts analogous to some parts of NRPSs are also used for production of modified amino acids (*e.g*. amino coumarin in *e.g*. novobiocin and β-hydroxy histidine as precursor for the imidazolone moiety in nikkomycin X) as building blocks for secondary metabolites. In bacteria and lower fungi biosynthetics genes required for production of secondary metabolites are typically clustered in one locus on the genome. In particular, in an embodiment wherein an NRPS is used, the NRPS may be a modular non-ribosomal peptide synthase comprising a lysine specific adenylation domain, a peptidyl carrier domain and a thioesterase/ cyclisation domain.

In a specific embodiment a biocatalyst for cyclisation of lysine to ACL can be found in a gene cluster encoding the biosynthesis of bengamides, nocardiamycins, capuramycins, circinatins or any other ACL or ACL-derivative containing secondary metabolite. Such a gene cluster may be present in any microorganism producing such a compound or a microbial endosymbiont thereof. Such a gene cluster can readily be identified by methods generally known in the art such as genome scanning, whole genome sequencing, PCR using degenerated primers, or Southern hybridisation using information from known biosynthetic pathways. A specific biocatalyst may consist of a truncated NRPS module consisting of an adenylation domain specific for the activation of lysine, a peptidyl carrier domain, and a specific cyclisation domain. This cyclisation domain is expected to be homologous to known thioesterases catalysing the macrocyclisation of cyclic non-ribosomal peptides such as *e.g*. tyrocidin. It is expected that a cyclisation domain specific for cyclisation of lysine contains specific signature motifs allowing its differentiation from other cyclising thioesterases or thioesterase domains. The domain required for cyclisation of lysine may be encoded by one open reading frame resulting in a modular biocatalyst or in separate open reading frames resulting in separate proteins, which together form the biocatalyst. In the present invention use of such a biocatalyst may be advantageous, since the reaction is coupled to the hydrolysis of ATP and thus (at least substantially) irreversible.

In an embodiment, ACL is prepared by chemically converting lysine.
This may for instance be accomplished by esterifying lysine with an alcohol, such as methanol, in the presence of thionyl chloride and neutralising the resultant reaction mixture with a base, such as sodium methoxide, whereby cyclisation occurs, *e.g*. as described in the Examples. It has been reported in Tetrahedron Lett. 1980, 21, 2443-2446 that L-lysine may be cyclised to form ACL, *e.g*. in refluxing toluene in the presence of a large excess of Al₂O₃. Alternatively, ACL may be formed by refluxing lysine and a hydroxide, such as NaOH, in a suitable alohol (for instance 1-propanol, 1-butanol, 1-pentanol or 1-hexanol), *e.g*. in equimolar amounts, optionally in the presence of an excess of Al₂O₃.

As mentioned above, in accordance with the invention ACL is converted into (*Z*)-6,7-dihydro-1*H*-azepin-2(5*H*)-one (6,7-DAO), which conversion is catalysed by a biocatalyst.

6,7-DAO may in particular be prepared from ACL in a method comprising biocatalytically removing the α-amino group from ACL by biocatalytic elimination of ammonia from ACL wherein a biocatalyst having ammonia lyase activity is used, thereby forming 6,7-DAO, or removal of the α-aminogroup from ACL by another biocatalyst able of catalysing such elimination or another biocatalyst able of catalysing such removal of the ammonia group.

Removal of the α-amino group of ACL to yield 6,7-DAO may in particular be catalysed by a biocatalyst comprising a lyase (EC 4). Preferably, a C-N lyase (EC 4.3) is used, more preferably an ammonia lyase (EC 4.3.1) is used.

A biocatalyst catalysing the conversion of ACL to 6,7-DAO may for instance originate from an organism, as mentioned above, when discussing biocatalysts in general.

It is also possible to select a suitable biocatalyst for the conversion of ACL to 6,7-DAO using a selection method, as described, next.

For instance, one may select for a biocatalyst using a library comprising a collection of potential biocatalysts for removal of the α-amino group from ACL. In a selection method for finding a suitable biocatalyst, the candidate biocatalysts are contacted with a culture medium wherein as a sole nitrogen source ACL and/or at least one functional analogue of ACL is present. Only those micro-organisms will be able to grow, which can use the ACL-analogue as a nitrogen source.

Thereafter, one or more samples are selected that show growth in such culture medium (the so called 'growing cultures'). Thereafter, one or more of these growing cultures are tested for having activity towards converting ACL to 6,7-DAO. Optionally, in particular in case only one or more ACL-analogues have been used as the sole nitrogen source, the growing cultures are first checked for showing activity towards converting the ACL-analogue or analogues, after which one or more cultures showing such activity are tested for their activity towards converting ACL to 6,7- DAO.

Thus, in a specific aspect of the invention, the invention relates to a method of finding a biocatalyst capable of catalysing the removal of the α-amino group from ACL, comprising
- providing a library comprising a plurality of candidate biocatalysts in one or more cell cultures, which cultures comprise a culture medium containing α-amino-ε-caprolactam and/or one or more analogues thereof as sole nitrogen source;
- selecting one or more candidate biocatalysts which grow in said culture medium; and
- screening for a biocatalyst which grows in said culture having catalytic activity in removing the α-amino group from ACL.

The term "selecting" as used herein is defined as a method in which one or more biocatalysts are tested for growth using certain specific conditions, which growth is an indication for the presence of the desired biocatalytic activity.

The term "screening" as used herein is defined as a method in which one or more biocatalysts are tested for one (or more) desired biocatalytic conversion(s).

The library may in particular be a metagenomic library, comprising genomic fragments of micro-organisms, which fragments may have been identified or which may be unidentified, and which fragments have been cloned into a suitable micro-organism for expression such as *Escherichia, Pseudomonas, Bacillus, Streptomyces* or *Saccharomyces*. The fragments may in principle originate from any organism and one or more organisms. The organism(s) may be culturable or unculturable under the existing conditions, may have a specific habitat, requiring specific environmental factors (*e.g*. temperature, pH, light, oxygen, nutrients) or symbiotic partners. In particular the organisms may be endosymbionts of a multicellular organism such as a sponge, insect, mammal or plant.

In an embodiment, the library comprises a variety of environmental samples containing candidate biocatalysts, in particular a variety of water samples (*e.g*. waste water samples), compost samples and/or soil samples. Such samples comprise a variety of wild-type micro-organisms.

The term "functional analogue of ACL" is used herein to indicate that the analogue comprises a functional group that may be recognised by the biocatalyst. In particular a functional analogue may have the L- or D- configuration or a mixture thereof in any ratio, consists of a seven-membered α-amino lactam or α-amino (thio)lactone with an additional carbon substituent at the α-position and optionally at the lactam nitrogen.

Preferably, an ACL-analogue is chosen which i) elicits the desired ACL ammonia lyase activity or alike activity leading to removal of the α-amino group from ACL and ii) has a low tendency towards eliciting side-reactions. In particular, the sole nitrogen source may consist of one or more compounds represented by formula I or II:

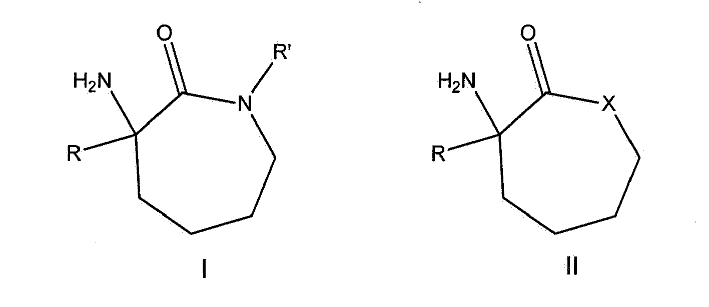

Herein, R and R' independently represent a hydrogen atom, or an organic moiety - which optionally comprises of one or more heteroatoms. Heteroatoms in the organic moieties R and R' may in particular be selected from N, S, O, F, Cl, Br, and I atoms. The organic moieties R and R' may in particular be independently selected from substituted and unsubstituted C1-C6 alkyl groups. X represents an O atom or an S atom.

The use of one or more functional analogues as the sole nitrogen source is preferred because the inventors have contemplated that the chance of finding a false positive would be higher when using ACL.

Another suitable selection method for finding a biocatalyst capable of catalysing the conversion of ACL to 6,7-DAO is based on lysine auxotrophy complementation. Herein a suitable host cell, which is lysine auxotroph, and which contains ACL-hydrolase activity is used for expression screening of genomic or metagenomic libraries. Such a host cell may be naturally occuring or can be engineered *e.g*. by inactivating the lysA gene in *E. coli* and expressing a suitable ACL-hydrolase. Such a host cell is then used for constructing a library as described above resulting in various host cells containing different DNA fragments. Various cells comprise different cloned genes.

The host cells are contacted with a culture medium comprising 6,7-DAO as sole lysine precursor. Then, one or more host cells are selected which grow in this medium. Thereafter, one or more growing host cells are usually tested for having catalytic activity for the conversion of 6,7-DAO to ACL. Thereafter one or more growing host cells (usually selected from those having catalytic activity for the conversion of 6,7-DAO to ACL) are tested for having catalytic activity for the conversion of ACL to 6,7-DAO. A host cell having such activity can be used as a biocatalyst, or be used to obtain a biocatalyst therefrom.

Accordingly, the invention further relates to a method of detecting a biocatalyst capable of catalysing the removal of the α-amino group of α-amino-ε-caprolactam, comprising
- providing lysine auxotrophic host cells, the host cells comprising a gene encoding an enzyme capable of catalysing the conversion of α-amino-ε-caprolactam into L-lysine, the host cells comprising a candidate gene encoding for an enzyme having lysine cyclase activity;
- contacting the host cells with a library comprising various vectors containing a candidate gene encoding for an enzyme capable of catalysing the conversion of 6,7-DAO to ACL, whereby at least a part of the host cells are provided with said vector;
- contacting the host cells, provided with said vector, with (*Z*)-6,7-dihydro-1*H*-azepin-2(5*H*)-one and an ammonia source;
- selecting one or more cultures which grow in said culture medium; and
- screening for one or more of said cultures which grow for having catalytic activity with respect to biocatalytic removal of the α-amino group of α-amino-ε-caprolactam, as the culture providing the biocatalyst capable of catalysing the elimination of the α-amino group of α-amino-ε-caprolactam.

Another suitable screening method contemplated by the inventors is based on using a molecular receptor and reporter system in a suitable host organism. Several such systems have been described in the art (Beggah, S.; Vogne, C.; Zenaro, E.; van der Meer, J. R. Microbial Biotechnology 2008, 1(1), 68-78; Sint Fiet, S.; van Beilen, J. B.; Witholt, B. Proceedings of the National Academy of Sciences 2006, 103(6), 1693-1698.). In such a system a suitable transcriptional regulator, herein also referred to as receptor, is able to bind a compound of interest such as 6,7-DAO or an analogue. Such a receptor may be a naturally occurring receptor having the desired properties in regards to *e.g*. specificity and binding affinity towards the compound of interest. In most cases these properties have to be optimized for the specific compound of interest and receptor interaction by protein engineering methods generally known in the art. Upon binding the receptor elicits transcription from a suitable promoter, which is linked to a suitable reporter gene, herein also referred to as reporter. Suitable reporters may in principle be a gene which elicits a detectibel, and preferably quantifiable, phenotype on the host strain such as production of a pigment, a fluorescent protein, an enzyme complementing an auxotrophy, or an antibiotic resistance marker.

Such a receptor/ reporter system may be established in a host and subsequently be used for screening (*e.g*. if using a fluorescent protein such as a green fluorescent protein as reporter) or selection (*e.g*. if using an antibiotic resistance gene as reporter) of a suitable biocatalyst for conversion of ACL to 6,7-DAO. The host cells are contacted with a culture medium comprising ACL or an analogue thereof. Then, one or more host cell cultures are selected or screened for which elicit a phenotype corresponding to the expression of the reporter. Thereafter, one or more such host cell cultures are usually tested for having catalytic activity for the conversion of ACL to 6,7-DAO. A host cell having such activity can be used as a biocatalyst, or be used to obtain a biocatalyst therefrom.

Accordingly, the invention also relates to a method of finding a biocatalyst capable of catalysing the removal of the α-amino group of α-amino ε-caprolactam, comprising
- identifying or engineering a receptor to specifically bind 6,7-DAO; - linking said receptor to a suitable reporter such as a β-galactosidase, green fluorescent protein, or an antibiotic resistance gene;
- optionally optimising the binding of 6,7-DAO to the receptor via one or more rounds of protein engineering to obtain desired specificity (i.e. no or low signal from the natural ligand and/ or ACL or analogues) and desired affinity towards 6,7-DAO or analogues thereof;
- expressing such a receptor/ reporter in a host suitable for metagenomic screening;
- contacting the host cells with a library comprising various vectors containing a candidate gene encoding for a biocatalyst (such as an enzyme) capable of catalysing the conversion of 6,7-DAO to ACL, whereby at least a part of the host cells comprise said vector;
- contacting the host cells, comprising said vector, with ACL or an analogue thereof;
- selecting or screening for one or more cultures which show the desired phenotype based on expression of the chosen reporter; and
- screening for one or more of said cultures for having catalytic activity with respect to biocatalytic removal of the α-amino group of α-amino-ε-caprolactam, as the culture providing the biocatalyst capable of catalysing the elimination of the α-amino group of α-amino-ε-caprolactam.

The gene encoding a biocatalyst, such as an enzyme, capable of catalysing the conversion of α-amino-ε-caprolactam into lysine may suitably be incorporated in the host cells using a vector, by conventional means.

The candidate gene encoding a biocatalyst having lysine cyclase activity may suitably be incorporated in the host cells using a vector, which may be the same or different as the vector encoding a biocatalyst capable of catalysing the conversion of α-amino-ε-caprolactam into lysine.

If desired, ε-caprolactam can be prepared by reducing the unsaturated carbon-carbon double bond of (*Z*)-6,7-dihydro-1*H*-azepin-2(5*H*)-one obtained in a method of the invention, yielding caprolactam.

Such reduction can be catalysed by a catalyst, capable of catalysing the reduction. In particular, a biocatalyst may be used for the reduction. Preferably such biocatalyst has reductase activity, in particular 6,7-DAO enone reductase activity, *i.e*. the catalyst is able to catalyse the reduction of the carbon-carbon double bond in 6,7-DAO, thereby forming caprolactam.

In particular, the biocatalyst may comprise an enzyme selected from the group of oxidoreductases (EC1), more in particular the oxidoreductase may be an oxidoreductase acting on the CH-CH group of donors (EC1.3) or an oxidoreductase that acts on NADH or NADPH (EC 1.6).

More specifically an oxidoreductase from EC 1.3.1 may be used, such as a 2-enone reductase (EC 1.3.1.33).

A specific example of class an EC 1.6 enzyme is old yellow enzyme 1 (OYE1) is EC 1.6.99.1. The biocatalyst for reducing 6,7-DAO may be used in combination with a cofactor, suitable cofactors are known in the art, depending on the biocatalyst (enzyme) that is used.

A biocatalyst capable of catalysing said reduction may originate from an organism such as mentioned above. In particular, said biocatalyst may originate from yeasts, plants, bacteria, fungi, archaea or mammals. More in particular a suitable biocatalyst capable of catalysing said reduction may originate from a micro-organism selected from *Candida macedoniensis, Kluyveromyces lactis, Pseudomonas fluorescens, Pseudomonas syringae* pv. *glycinea, Escherichia coli, Saccharomyces cerevisiae* and *Bacillus subtilis*.

In a specific embodiment, a biocatalyst comprising an amino acid sequence represented by Sequence ID 2, 4, 6, 8, 10, 12, 14 or a homologue thereof is used for catalysing the reduction of 6,7-DAO.

Reaction conditions may be chosen depending upon known conditions for the biocatalyst, in particular the enzyme, the information disclosed herein and optionally some routine experimentation.

In principle, the pH of the reaction medium used may be chosen within wide limits, as long as the biocatalyst is active under the pH conditions. Alkaline, neutral or acidic conditions may be used, depending on the biocatalyst and other factors. In case the method includes the use of a micro-organism, *e.g*. for expressing an enzyme catalysing a method of the invention, the pH is selected such that the micro-organism is capable of performing its intended function or functions. The pH may in particular be chosen within the range of four pH units below neutral pH and two pH units above neutral pH, *i.e*. between pH 3 and pH 9 in case of an essentially aqueous system at 25 °C. A system is considered aqueous if water is the only solvent or the predominant solvent (> 50 wt. %, in particular > 90 wt. %, based on total liquids), wherein *e.g*. a minor amount of alcohol or another solvent (< 50 wt. %, in particular < 10 wt. %, based on total liquids) may be dissolved (*e.g*. as a carbon source) in such a concentration that micro-organisms which may be present remain active. In particular in case a yeast and/or a fungus is used, acidic conditions may be preferred, in particular the pH may be in the range of pH 3 to pH 8, based on an essentially aqueous system at 25 °C. If desired, the pH may be adjusted using an acid and/or a base or buffered with a suitable combination of an acid and a base.

In principle, the incubation conditions can be chosen within wide limits as long as the biocatalyst shows sufficient activity and/ or growth. Conditions may be selected from the group of aerobic, oxygen limited and anaerobic conditions.

Anaerobic conditions are herein defined as conditions without any oxygen or in which substantially no oxygen is consumed by the biocatalyst, in particular a micro-organism, and usually corresponds to an oxygen consumption of less than 5 mmol/l.h, in particular to an oxygen consumption of less than 2.5 mmol/l.h, or less than 1 mmol/l.h.

Aerobic conditions are conditions in which a sufficient level of oxygen for unrestricted growth is dissolved in the medium, able to support a rate of oxygen consumption of at least 10 mmol/l.h, more preferably more than 20 mmol/l.h, even more preferably more than 50 mmol/l.h, and most preferably more than 100 mmol/l.h.

Oxygen-limited conditions are defined as conditions in which the oxygen consumption is limited by the oxygen transfer from the gas to the liquid. The lower limit for oxygen-limited conditions is determined by the upper limit for anaerobic conditions, *i*.*e*. usually at least 1 mmol/l.h, and in particular at least 2.5 mmol/l.h, or most specifically at least 5 mmol/l.h. The upper limit for oxygen-limited conditions is determined by the lower limit for aerobic conditions, *i.e.* less than 100 mmol/l.h, less than 50 mmol/l.h, less than 20 mmol/l.h, or less than to 10 mmol/l.h.

Whether conditions are aerobic, anaerobic or oxygen limited is dependent on the conditions under which the method is carried out, in particular by the amount and composition of ingoing gas flow, the actual mixing/mass transfer properties of the equipment used, the type of micro-organism used and the micro-organism density.

In principle, the temperature used is not critical, as long as the biocatalyst, in particular the enzyme, shows substantial activity. Generally, the temperature may be at least 0 °C, in particular at least 15 °C, more in particular at least 20 °C. A desired maximum temperature depends upon the biocatalyst. In general such maximum temperature is known in the art, *e.g*. indicated in a product data sheet in case of a commercially available biocatalyst, or can be determined routinely based on common general knowledge and the information disclosed herein. The temperature is usually 90 °C or less, preferably 70 °C or less, in particular 50 °C or less, more in particular or 40 °C or less.

In particular if a biocatalytic reaction is performed outside a host organism, a reaction medium comprising an organic solvent may be used in a high concentration (*e.g*. more than 50 wt. %, or more than 90 wt. %, based on total liquids), in case an enzyme is used that retains sufficient activity in such a medium.

In an advantageous method caprolactam is prepared making use of a whole cell biotransformation of the substrate for caprolactam or an intermediate for forming caprolactam (ACL, 6,7-DAO), comprising a micro-organism wherein a lysine cyclase, and an ammonia lyase and/or biocatalyst with activity for removal the α-amino group from ACL, and a 6,7-DAO enone reductase and/or other biocatalyst capable of reducing 6,7-DAO to caprolactam are produced, and a carbon source for the micro-organism.

The carbon source may in particular contain at least one compound selected from the group of monohydric alcohols, polyhydric alcohols, carboxylic acids, carbon dioxide, fatty acids, glycerides, including mixtures comprising any of said compounds. Suitable monohydric alcohols include methanol and ethanol. Suitable polyols include glycerol and carbohydrates. Suitable fatty acids or glycerides may in particular be provided in the form of an edible oil, preferably of plant origin.

In particular a carbohydrate may be used, because usually carbohydrates can be obtained in large amounts from a biologically renewable source, such as an agricultural product, preferably an agricultural waste-material. Preferably a carbohydrate is used selected from the group of glucose, fructose, sucrose, lactose, saccharose, starch, cellulose and hemi-cellulose. Particularly preferred are glucose, oligosaccharides comprising glucose and polysaccharides comprising glucose.

A cell comprising one or more enzymes for catalysing a reaction step in a method of the invention can be constructed using molecular biological techniques, which are known in the art *per se*. For instance, if one or more biocatalysts are to be produced in a heterologous system, such techniques can be used to provide a vector which comprises one or more genes encoding one or more of said biocatalysts. One or more vectors may be used which each comprise one or more genes. A vector comprising one or more of such genes can comprise one or more regulatory elements, *e.g*. one or more promoters, which may be operably linked to a gene encoding a biocatalyst.

As used herein, the term "operably linked" refers to a linkage of polynucleotide elements (or coding sequences or nucleic acid sequence) in a functional relationship. A nucleic acid sequence is "operably linked" when it is placed into a functional relationship with another nucleic acid sequence. For instance, a promoter or enhancer is operably linked to a coding sequence if it affects the transcription of the coding sequence.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skilled in the art to act directly or indirectly to regulate the amount of transcription from the promoter. A "constitutive" promoter is a promoter that is active under most environmental and developmental conditions. An "inducible" promoter is a promoter that is active under environmental or developmental regulation. The term "homologous" when used to indicate the relation between a given (recombinant) nucleic acid or polypeptide molecule and a given host organism or host cell, is understood to mean that in nature the nucleic acid or polypeptide molecule is produced by a host cell or organisms of the same species, preferably of the same variety or strain.

The promoter that could be used to achieve the expression of the nucleotide sequences coding for a biocatalyst for use in a method of the invention, in particular an ammonia lyase, and optionally at least one biocatalyst selected from the group of lysine cyclases and 6,7-DAO enone reductases, such as described herein above may be native to the nucleotide sequence coding for the biocatalyst to be expressed, or may be heterologous to the nucleotide sequence (coding sequence) to which it is operably linked. Preferably, the promoter is homologous, *i.e.* endogenous to the host cell.

If a heterologous promoter (to the nucleotide sequence encoding for the biocatalyst of interest) is used, the heterologous promoter is preferably capable of producing a higher steady state level of the transcript comprising the coding sequence (or is capable of producing more transcript molecules, *i.e.* mRNA molecules, per unit of time) than is the promoter that is native to the coding sequence. Suitable promoters in this context include both constitutive and inducible natural promoters as well as engineered promoters, which are known to the person skilled in the art.

A "strong constitutive promoter" is one which causes mRNAs to be initiated at high frequency compared to a native host cell. Examples of such strong constitutive promoters in Gram-positive micro-organisms include SP01-26, SP01-15, *veg,* pyc (pyruvate carboxylase promoter), and *amy*E.

Examples of inducible promoters in Gram-positive micro-organisms include, the IPTG inducible Pspac promoter, the xylose inducible PxylA promoter.

Examples of constitutive and inducible promoters in Gram-negative microorganisms include, but are not limited to, *tac, tet, trp-tet, lpp, lac, lpp-lac, laclq, T7, T5, T3, gal, trc, ara* (*P_{BAD}*)*,* SP6, λ-P_{R}, and λ-P_{L}.

Promoters for (filamentous) fungal cells are known in the art and can be, for example, the glucose-6-phosphate dehydrogenase *gpd*A promoters, protease promoters such as *pep*A, *pep*B, *pep*C, the glucoamylase *gla*A promoters, amylase *amy*A, *amy*B promoters, the catalase *cat*R or catA promoters, glucose oxidase *gox*C promoter, beta-galactosidase *lac*A promoter, alpha-glucosidase *agl*A promoter, translation elongation factor *tef*A promoter, xylanase promoters such as *xln*A*, xln*B*, xln*C, *xln*D, cellulase promoters such as *egl*A, *egl*B, *cbh*A, promoters of transcriptional regulators such as *are*A, *cre*A, *xln*R, *pac*C, *prt*T, etc or any other, and can be found among others at the NCBI website (http://www.ncbi.nlm.nih.gov/entrez/).

The term "heterologous" when used with respect to a nucleic acid (DNA or RNA) or protein refers to a nucleic acid or protein that does not occur naturally as part of the organism, cell, genome or DNA or RNA sequence in which it is present, or that is found in a cell or location or locations in the genome or DNA or RNA sequence that differ from that in which it is found in nature. Heterologous nucleic acids or proteins are not endogenous to the cell into which it is introduced, but has been obtained from another cell or synthetically or recombinantly produced. Generally, though not necessarily, such nucleic acids encode proteins that are not normally produced by the cell in which the DNA is transcribed or expressed. Similarly exogenous RNA encodes for proteins not normally expressed in the cell in which the exogenous RNA is present. Heterologous nucleic acids and proteins may also be referred to as foreign nucleic acids or proteins. Any nucleic acid or protein that one of skill in the art would recognize as heterologous or foreign to the cell in which it is expressed is herein encompassed by the term heterologous nucleic acid or protein.

A method according to the invention may be carried out in a host organism. Accordingly, the invention also relates to a novel host cell comprising one or more biocatalysts capable of catalysing the conversion of ACL to 6,7-DAO. The invention also relates to a novel vector comprising one or more genes encoding one or more of such biocatalysts (in particular enzymes) and to a novel host cell comprising one or more vectors comprising one or more genes encoding one or more of such biocatalysts (in particular enzymes).

One or more suitable genes may in particular be selected amongst genes encoding a biocatalyst (such as an enzyme) as mentioned herein above.

A host cell according to the invention comprises at least one recombinant vector comprising a nucleic acid sequence encoding a biocatalyst (in particular an enzyme) with ammonia lyase activity. Optionally the cell comprises a nucleic acid sequence encoding a biocatalyst (in particular an enzyme) with lysine cyclase activity. In a specific embodiment a recombinant vector comprising a nucleic acid sequence encoding a biocatalyst (in particular an enzyme) with lysine cyclase activity is present, which sequence can be in the same or a different vector as the sequence encoding the biocatalyst having ammonia lyase activity.

In an embodiment a host cell according to the invention comprises at least one recombinant vector comprising a nucleic acid sequence encoding a biocatalyst (in particular an enzyme) with ACL ammonia lyase activity. Optionally, the cell comprises a nucleic acid sequence encoding a biocatalyst (in particular an enzyme) with 6,7-DAO enone reductase activity, which sequences can be in the same or a different vector. In a specific embodiment a recombinant vector comprising a nucleic acid sequence encoding a biocatalyst (in particular an enzyme) with 6,7-DAO enone reductase activity is present, which sequence can be in the same or a different vector as the sequence encoding the biocatalyst having ACL ammonia lyase activity.

Optionally, the cell comprises a nucleic acid sequence encoding a biocatalyst (in particular an enzyme) with 6,7-DAO enone reductase activity. In a specific embodiment a recombinant vector comprising a nucleic acid sequence encoding a biocatalyst (in particular an enzyme) with 6,7-DAO enone reductase activity is present, which sequence can be in the same or a different vector as the sequence encoding the biocatalyst ACL ammonia lyase activity. In a specific embodiment, the cell comprises a nucleic acid sequence encoding a biocatalyst comprising an amino acid sequence represented by Sequence ID 2, 4, 6, 8, 10, 12, 14 or a homologue thereof. Possible nucleic acid sequences encoding said sequences are shown in Sequence ID 1, 3, 5, 7, 9, 11 and 13, respectively. Preferred sequences include the nucleic acid sequences selected from the group of Sequence ID 15-18 and non-wild type functional analogues thereof. Functional analogues are in particular sequences encoding the same amino acid sequence having a similar or better level of expression in a host cell of interest.

A cell of the invention comprising a nucleic acid sequence encoding a biocatalyst with ammonia lyase activity, a nucleic acid sequence encoding a biocatalyst with lysine cyclase activity and a nucleic acid sequence encoding a biocatalyst with 6,7-DAO enone reductase activity, is particularly suitable for a method wherein caprolactam is prepared from lysine, wherein purely chemical (*i.e*. not biocatalysed) reaction steps are avoided are at least considerably reduced. Thus, the cell may be used as a biocatalyst for all reaction steps to prepare caprolactam from lysine, which steps may take place intracellularly in at least some embodiments. Such as cell may be a natural micro-organism or a recombinant organism. In the recombinant organism at least one, at least two or at least three recombinant nucleic acid sequences are present for encoding any of said biocatalysts (usually enzymes).

The host cell may for instance be selected from bacteria, yeasts or fungi. In particular from the genera selected from the group of *Aspergillus, Penicillium, Saccharomyces, Kluyveromyces, Pichia, Candida, Hansenula, Bacillus, Corynebacterium*, and *Escherichia*, in which one or more encoding nucleic acid sequences as mentioned above have been cloned and expressed. In particular, the host cell may be selected from the group of *Escherichia coli, Bacillus subtilis, Corynebacterium glutamicum, Aspergillus niger, Penicillium chrysogenum, Saccharomyces cervisiae, Hansenula polymorpha, Candida albicans, Kluyveromyces lactis, Pichia stipitis* and *Pichia pastoris* host cells. In a preferred embodiment, the host cell is capable of producing lysine (as a precursor).

The host cell may be in principle a naturally occurring organism or may be an engineered organism. Such an organism can be engineered using a mutation screening or metabolic engineering strategies known in the art. For instance such a host cell may be selected of the genus *Corynebacterium*, in particular *C*. *glutamicum*, enteric bacteria, in particular *Escherichia coli, Bacillus*, in particular *B*. *subtilis* and *B. methanolicus*, and *Saccharomyces*, in particular *S*. *cerevisiae*. Especially preferred are *C*. *glutamicum* or *B*. *methanolicus* strains which have been developed for the industrial production of lysine.

## Claims

1. Method for preparing (*Z*)-6,7-dihydro-1*H*-azepin-2(5*H*)-one comprising removing the α-amino group of α-amino-ε-caprolactam, wherein the removal is catalysed by a biocatalyst.

2. Method according to claim 1, wherein the biocatalyst comprises a C-N lyase (EC 4.3).

3. Method according to claim 2, wherein the biocatalyst comprises an ammonia lyase (EC 4.3.1).

4. Method according to any of the preceding claims, wherein the α-amino-ε-caprolactam has been prepared by converting lysine.

5. Method for preparing ε-caprolactam comprising reducing the carbon-carbon double bond of (*Z*)-6,7-dihydro-1*H*-azepin-2(5*H*)-one prepared in a method according to any of the preceding claims.

6. Method according to claim 5, wherein the reduction is catalysed by an (*Z*)-6,7-dihydro-1*H*-azepin-2(5*H*)-one enone reductase.

7. Method according to claim 6, wherein said enone reductase comprises an amino acid sequence represented by Sequence ID 2, 4, 6, 8, 10, 12, 14 or a homologue of any of these.

8. A host cell comprising at least one recombinant vector comprising a nucleic acid sequence encoding a biocatalyst with α-amino-ε-caprolactam ammonia lyase activity.

9. A host cell according to claim 8, comprising a nucleic acid sequence encoding a biocatalyst with (*Z*)-6,7-dihydro-1*H*-azepin-2(5*H*)-one enone reductase activity.

10. A host cell according to claim 9, wherein the nucleic acid sequence encoding the biocatalyst with (*Z*)-6,7-dihydro-1*H*-azepin-2(5*H*)-one enone reductase activity comprises a nucleic acid sequence according to Sequence ID 1, 3, 5, 7, 9, 11, 13, 15, 16, 17 or 18.

11. Host cell according to claim 8, 9 or 10, wherein the host cell is selected from the group of genera consisting of *Aspergillus, Penicillium, Saccharomyces, Kluyveromyces, Pichia, Candida, Hansenula, Bacillus, Corynebacterium* and *Escherichia.*
